(19)

Europäisches
Patentamt
European
Patent Office
Office européen
des brevets

(11) **EP 2 316 372 A1**

(12) **EUROPEAN PATENT APPLICATION**

(43) Date of publication:
**04.05.2011 Bulletin 2011/18**

(51) Int Cl.:
*A61B 18/20* (2006.01)

(21) Application number: **10012155.7**

(22) Date of filing: **25.01.2001**

(84) Designated Contracting States:
**AT BE CH CY DE DK ES FI FR GB GR IE IT LI LU
MC NL PT SE TR**

(30) Priority: **25.01.2000 US 177943 P
27.09.2000 US 235814 P**

(62) Document number(s) of the earlier application(s) in
accordance with Art. 76 EPC:
**01906689.3 / 1 251 791**

(71) Applicant: **Palomar Medical Technologies, Inc.
Burlington, MA 01803 (US)**

(72) Inventors:
• **Altshuler, Gregory B.
Wilmington
MA 01887 (US)**
• **Anderson, Rox R.
Lexington
MA 02173 (US)**

• **Battle, Eliot
Boston
MA 02114 (US)**
• **Smotrich, Michael
Burlington
MA 01803 (US)**
• **Zenzie, Henry M.
Dover, MA 02030 (US)**
• **Manstein, Dieter
Boston
MA 02114 (US)**

(74) Representative: **Lang, Johannes et al
Bardehle Pagenberg
Galileiplatz 1
81679 München (DE)**

Remarks:
This application was filed on 30-09-2010 as a
divisional application to the application mentioned
under INID code 62.

(54) **Method and apparatus for medical treatment utilizing long duration electromagnetic radiation**

(57)     A method and apparatus are provided for performing a medical procedure on a patient, for example a dermatological procedure, by use of electromagnetic radiation (EMR) having a relatively low peak power, and in particular a peak power low enough so as not to result in a phase change in the heater or chromophore absorbing radiation which would result in a significant reduction in its absorption, and of relatively long duration which is generally greater than, sometimes significantly greater than, the thermal relaxation time of the irradiated target.

FIG. 1

EP 2 316 372 A1

## Description

### Field of the Invention

This invention relates to methods and apparatus for medical treatments using electromagnetic radiation, and more particularly to such methods and apparatus for treatment where the electromagnetic radiation is applied to a treatment area for a relatively long time interval at relatively low peak power.

### Related Applications

This application relates to Serial No. 60/177,943, filed January 25, 2000 and Serial No. 60/235,814, filed September 27, 2000.

### Background

For many years, electromagnetic radiation (EMR) from lasers, lamps and other sources, including microwave and radio frequency (RF) has been used to treat a variety of medical conditions in ophthalmology, dermatology, urology, otolaryngology and other areas. For example in dermatology, hair removal, treatment of various pigmented lesions, removal of unwanted vessels, skin resurfacing and the like are current applications. For all of these treatments, a chromophore, which may be naturally existing in the patient's body or may be introduced into the body, absorbs at least some wavelength or wavelengths of the applied EMR and is heated as a result of this absorption. A natural chromophore can for example be water, melanin, hemoglobin, protein, or lipid. An artificial chromophore can for example be dye, ink, carbon particles or magnetic particles. The heating of the chromophore usually results in the destruction of an unwanted hair follicle, pigmented lesion, tattoo, blood vessel, etc. to effect the desired treatment.

However, all existing methods and apparatus for optical dermatology present problems. First, in most cases, in order to achieve the desired dermatological effect, substantial energy must be applied to the skin component being treated. Heretofore, in order to get sufficient energy to the treated component, it has been necessary to use a relatively high peak power optical source, with for example one or more kilowatts of peak power generally being required in order to achieve long term hair removal. However, since the optical radiation must pass through the patient's epidermis to reach the treatment area, and since the epidermis contains melanin which is a chromophore at the wavelengths typically used for hair removal and certain other treatments, such high power applied to the skin can result in epidermal damage. While various techniques for cooling the epidermis during treatment have been discussed in the prior art, and these techniques do raise the energy threshold at which thermal damage occurs, it has been found that, while these techniques are effective for lighter skinned patients, for example patients having skin with lower Fitzpatrick skin type classification numbers, they frequently do not provide sufficient protection when treating darker skinned patients having, for example, Fitzpatrick skin types III-VI, and particularly for dark skinned individuals having skin types V and VI or tanned patients. This has meant that the benefits of optical dermatology treatments have heretofore not been available to a significant percentage of the population, and has limited the ability to treat exposed, and thus frequently tanned, parts of a patient's body, which are the parts on which treatment is most frequently desired.

Second, the requirement to generate high peak power has required the use of large, and relatively expensive, lasers and other optical sources. For example, to generate the requisite peak power using diode lasers, a laser head having as many as 100 diode bars may be required, depending to some extent on wavelength. In addition to the cost of the diode bars themselves, the use of such a large number of diode bars creates serious thermal management problems, further complicating the design and increasing the cost of the resulting diode laser device.

In addition, existing systems, which tend to apply energy to the skin component being treated over a relatively short time interval, can result in the explosion of the chromophore absorbing the heat, and thus in unwanted side effects, for example unsightly skin purpura, and in the generation of water vapor which may interfere with the efficient transfer of energy and/or heat to the component being treated. The process by which living tissues undergo thermal damage, including coagulative necrosis, is described in the scientific literature known to those skilled in the art, and will not be described here in any detail. High peak temperature of the chromophore can also cause chemical reactions which result in the formation of unwanted chemical substances. These substances can have a bad smell, this being a particular problem for hair removal, or can cause harm to the patient as a result of unpredictable effects of these substances on the body.

The use of high peak power lasers or other EMR sources also results in dermatology treatments using such apparatus being dangerous in that improper operation of the equipment can cause potentially serious injury to a patient's skin, eyes, or other portions of the patient's body, and if not carefully operated, can also cause injury to the person operating the equipment. This has resulted in a requirement that most such equipment be operated either by a dermatologist or other physician or by a skilled professional under the supervision of a doctor. The high cost of the equipment coupled with the requirement for treatments to be performed by skilled professionals has resulted in optical or other EMR dermatology procedures being relatively expensive. This, coupled with the fact that most EMR dermatology procedures are not covered by health insurance, has significantly limited the availability of such treatments to the general public. Therefore, treatment procedures which both significantly reduce the cost of the equipment required and permit treatments to be performed by less highly trained personnel could significantly reduce the cost and enhance the availability of optical dermatology procedures to the general public.

It is also desirable to use feedback to control some EMR medical procedures, a patient parameter, for example skin temperature, being detected and utilized to control EMR energy, cooling and/or other treatment parameter, or if appropriate, to terminate treatment. However, feedback control is not practical with pulses of less than several ms, and works best with even longer pulse durations, in the order of 100 ms or greater. Feedback is another technique which permits less trained personnel to safely operate the equipment.

Further, for the EMR dermatology treatments discussed above, the chromophore absorbing radiation and being heated thereby and the target to be acted on for destruction or other treatment generally occupying the same area, the chromophore being the treatment target. However, there are applications in EMR dermatology where the treatment target is not a chromophore and a chromophore does not exist in the immediate area of the treatment target; or in another words where non-uniform absorption exists in the target area, part of the target area exhibiting weak absorption or no absorption at all, while other parts show significant absorption. In these cases, damage to weakly absorbing/ nonabsorbing portions can only be achieved by heat diffusion from highly pigmented, strongly absorbing portions of a target area, such portions frequently being referred to hereinafter as "heater portions" or simply as "heaters". However, standard theories heretofore used for developing EMR radiation treatments, including the theory of selective photothermolysis, are only applicable where the chromophore and destruction target occupy substantially the same space and do not provide a reliable basis for developing treatment protocols for target areas having non-uniform absorption characteristics, and in particular where the destruction target has little or no absorption at the applied wavelength(s) and is spaced from any heater portion.

Finally, there are EMR treatments where enough energy cannot be applied to the target during a single treatment to effect the desired therapeutic result without undesired side effects, for example epidermal damage. However, with standard EMR treatments, the chromophore is generally destroyed during the treatment, so that weeks or even months must pass before the chromophore regenerates sufficiently for another treatment to be performed. It would be far preferable if two or more EMR treatments could be performed during a single treatment session, or at least within days of each other, rather then within weeks or months.

Many of the problems indicated above for dermatology can also arise when performing other EMR medical procedures, including the need to protect the patient's epidermis and treatment of targets having non-uniform absorption.

A need therefore exists for an improved method and apparatus for performing optical dermatology and other EMR medical procedures which overcomes the various problems indicated above, and in particular which facilitates treatment of non-uniformly absorbing areas and of dark skinned/tanned patients, which is both less expensive and safer than existing procedures, which permits multiple treatments during a single session, or closely spaced sessions, aud which facilitates feedback control of treatments.

## Summary of the Invention

In accordance with the above, this invention provides a method and apparatus for performing a medical procedure on a treatment area of a patient's body, which procedure may, for example, be a dermatology procedure such as hair removal, treatment of vascular lesions or collagen restructuring for wrinkle removal or other purposes. The method and apparatus involve applying electromagnetic radiation (EMR) of an appropriate wavelength from a source of such EMR through a suitable head to the treatment area. For preferred embodiments, the EMR is optical radiation, for example, coherent optical radiation from a laser or non-coherent optical radiation from a flash lamp, filament lamp, LED or other suitable source. Key features of the invention are the power profile for the applied radiation and the duration for application of radiation to the target area.

In accordance with one aspect of the invention, the power profile for the applied radiation has an average power ($P_a$) sufficient to effect the desired result over the application duration and a peak power ($P_p$) which does not result in destruction of the chromophore or heater. The duration of radiation application to the target area is longer than the thermal relaxation time (TRT) of the target area, being long enough for sufficient energy for the procedure to be applied at the power profile to the treatment area. For this embodiment, the duration of radiation application can be up to 20 seconds and more, particularly for hyperthermia treatments, and $P_p$ is preferably less than 500 watts. For some continuous wave embodiments of the invention, $P_p$ and $P_a$ for the radiation over the duration of application are roughly the same.

For this invention:

■ The EMR wavelength should be chosen to maximize contrast between the absorption coefficient of the pigmented area or heater and that of the tissue surrounding the target area. This postulate is identical to the case of classical selective photothermolysis.

■ The EMR power should be limited to prevent the pigmented heater area from destruction or from otherwise losing the ability to absorb, but must be sufficient to achieve a heater temperature higher than the target damage temperature.

■ The pulsewidth should be made shorter than or equal to the thermal damage time (TDT), which can be significantly longer than the thermal relaxation time (TRT) of the target. TDT is the time required for irreversible target damage with sparing of the surrounding tissue.

For some embodiments, irradiation is applied to heat at least one chromophore in the treatment area and the peak power is selected so as to heat the at least one chromophore only to a selected temperature, which temperature is below that at which the chromophore undergoes a change which results in significant lose of absorption at the applied wavelength or wavelengths. For example, the temperature to which the chromophore is heated may be no more than approximately 100-110° C so as to avoid vaporization of water in the tissue. For some embodiments, the procedure is hair removal, the chromophore is melanin within a hair shaft of the hair follicle and the duration of radiation application is sufficient for heat transfer from the hair shaft to the outer root sheath of the follicle to damage stem cells located thereat. The chromophore may also be another chromophore either located within the hair follicle or introduced therein which results in the power profile and duration being sufficient to cause destruction of stem cells at an outer root sheath of the follicle, a papilla for the follicle and/or other critical follicle cells.

For another embodiment, the procedure is wrinkle removal, the chromophore is melanin at the dermis/epidermis (DE) junction, and the power profile and duration are sufficient to heat collagen in the papillary dermis sufficiently to restructure such collagen. For still other embodiments, the procedure is destruction of vascular lesions, the chromophore being at least hemoglobin or water of the blood in the vascular lesion and the duration being sufficiently longer at the power profile for heating and denaturation of the wall of the vessel. Water may also be the chromophore for various other treatments, including treatments for skin rejuvenation or wrinlde removal.

In accordance with still another aspect of the invention, the wavelength of the EMR from the source is absorbed by melanin in the patient's epidermis, including at the dermis/epidermis (DE) junction, and the power profile and duration are such that heat generated as a result of EMR absorption in epidermal melanin can migrate to the skin surface and be removed concurrent with irradiation, thereby controlling temperature increase in the epidermis during irradiation. In accordance with still another aspect of the invention, the treatment area has a target area with a thermal relaxation time and the duration is longer than this thermal relaxation time. In particular, the target area includes a heater part or portion which is highly absorbing at the wavelength(s) of the EMR source and the EMR radiation is applied at least to such heater portion of the target area for a duration significantly greater than the thermal relaxation time of the target area. The target area also has a thermal damage time (TDT) which is the time required at the applied power profile for the entire target area to reach a thermal destruction temperature, and the duration of the optical radiation is substantially equal to such TDT.

More specifically, the duration of the optical radiation (T) is equal $(TDT-\delta)$ where $\delta$ is roughly the propagation time for the front of the heat from the heater portion to a non-heater portion of the target furthest from the heater. TDT and TRT may be related such that $TDT = TRT_T(x, \Delta)$, where x is a geometrical factor and $\Delta$ is a temperature factor. More specifically, $x = d_2/d_1$, where $d_1$ = size of heater portion and $d_2$ = size of total target; and $\Delta = (T_2-T_0)/(T_1-T_0)$ where $T_0$ is target and heater temperature before irradiation, $T_1$ is the heater temperature and $T_2$ is the temperature at which irreversible thermal damage of the target area generally occurs. Where the target is a hair follicle, and the heater includes a pigmented hair shaft or hair matrix in the follicle, TDT is the time required for heat to the thermal destruction temperature to reach an outer sheath of the follicle. Similarly, where the target is a blood vessel, and the heater includes blood in the blood vessel, TDT is the time required for heat to the thermal destruction temperature to reach through walls of the blood vessel in the target area. Where the target is collagen in the papillary dermis, and the heater is melanin at the DE junction, TDT is the time required for heat at the thermal destruction temperature for collagen to reach a desired depth in the papillary dermis. For substantially all applications, the power profile should be such that, at TDT, the entire target area is at a temperature of at least the thermal destruction temperature, but substantially no tissue outside the target area is at or above the thermal destruction temperature.

To maximize energy or power delivered to a target inside a patient's body, it is generally desirable to cool the patient's skin in an area over the target area to remove heat from at least the patient's epidermis. Such cooling may be active cooling, for example having a chilled plate, lens, waveguide or the like in contact with the patient's skin, applying a cryogen spray to the patient's skin, gas/liquid flow across skin surface or other known contact or non-contact active cooling techniques, or may be passive, relying on heat diffusion from the skin surface.

In accordance with another aspect of the invention, the power profile of applied radiation results in heating of the heater to a temperature T which is greater than the thermal destruction temperature for at least a portion of the target to be damaged, but less than a collapse temperature at which the heater portion undergoes a change which results in significant loss of absorption at the at least one wavelength. The heater part of the target area may be a naturally-occurring chromophore in the patient's body, or may be an artificial chromophore introduced to the target area. Where the medical procedure is hair removal, the heater may include a hair shaft or hair matrix in the hair follicle which has a collapse temperature of up to 250° C, the radiation duration in this case being up to approximately 20 seconds. Where the medical procedure is removal of vascular lesions or collagen remodeling, the duration of radiation application may, for example, also be up to approximately 20 seconds.

In accordance with still another aspect of the invention, the target area includes at least one highly absorbent heater portion and at least one non-heater portion having weak absorption to no absorption, the at least one non-heater portion being spaced to varying degrees from the at least one heater portion. Electromagnetic radiation from a suitable source is applied to at least the heater portion(s) of the target area, the radiation including at least one wavelength highly absorbed by the heater portion(s) and having a power profile which heats the heater portion(s) to a temperature T greater than the thermal damage temperature for the portion(s) of the target area to be damaged, but less than the collapse temperature at which heater portion(s) undergo a change which results in significant loss of absorption at the applied at least one wavelength, the radiation being applied for a duration sufficient with the power profile to permit heating of substantially all of the target area from the heater portions to a temperature sufficient to accomplish the medical procedure. Where there are a plurality of target areas in an aperture being irradiated by the optical radiation, each of which areas is of a size $d_2$, and the centers of the target areas are spaced by a distance $d_3$, the ratio of fluence $F_{NS}$ at which damage outside the target areas occur to fluence $F_s$ at which selective damage of a complete target area occurs is $F_{NS}/F_s = (d_3/d_2)^n$, where n is dependent on target shape. The ratio $F_{NS}/F_s$ has been found to drop significantly for $Y=d_3/d_2 < 5$.

In accordance with still another aspect of the invention, the wavelength utilized, in addition to being appropriate for the medical procedure being performed, is also absorbed by melanin in the patient's epidermis, including at the DE junction. In this case, the EMR has a power profile and duration which are sufficient to effect the medical procedure and are such that heat generated as a result of EMR absorption in the epidermal melanin can migrate to the skin surface and be removed concurrent with irradiation, thereby controlling temperature increase in the epidermis during irradiation. Active cooling may be applied to the skin surface to facilitate the removal of heat therefrom.

In accordance with still another aspect of the invention, the power profile is such that the temperature of the heater is maintained substantially constant for the duration of EMR application. This power profile may for example result in an optical power which decreases substantially exponentially for the EMR duration.

The foregoing and other objects, features and advantages of the invention will be apparent in the following more particular description of preferred embodiments of the invention as illustrated in the accompanying drawings.

**Brief Description of the Drawings**

Fig. 1 is a schematic representation of a portion of a patient's skin and of a system suitable for practicing the teachings of this invention positioned adjacent thereto;

Fig. 2 is a diagrammatic representation of a biological target with a spaced chromophore/heater and target area;

Figs. 3 a and 3b are diagrams illustrating optical power versus time and temperature versus time respectively for a rectangular EMR pulse mode, while Figs. 3c and 3d are diagrams illustrating power versus time and temperature versus time for a rectangular temperature pulse mode respectively;

Figs. 4a-4c diagrammatically illustrate three geometrical shapes, namely, plane, cylindrical and spherical, respectively, utilized in various examples;

Figs. 5a and 5b are diagrams illustrating temperature versus location for the rectangular EMR pulse mode and rectangular temperature pulse mode, respectively, at different times, Fig. 5c being a cross-section through an illustrative hair follicle;

Figs. 6a-6c are diagrams showing the ratio of r (x, $\Delta$) = TDT/TRT as a function of a geometrical factor x for two heating modes for a plane target, cylindrical target and spherical target, respectively;

Fig. 7 is a diagram of temperature versus position relative to a hair shaft for a cylindrical target for different temperatures of the hair shaft;

Fig. 8 is a chart showing calculated temperature distribution as a function of depth for a cooled surface (10°C) and a laser pulse duration of 1 second at roughly 150 J/cm$^2$;

Figs. 9a-9d are charts illustrating temperature versus depth for pulse durations of 3 ms, 10 ms, 100 ms and 300 ms, respectively;

Figs. 10a-10c are diagrams illustrating the dependence of pulse width to hair shaft diameter for various densities of hairs;

Fig. 11 is a diagram illustrating the relationship of pulse power and duration for various hair and skin types;

Fig. 12 is a diagrammatic representation of an ideal model for cylindrical targets with equal spacing; and

Figs. 13a and 13b are diagrams illustrating the dependence of thermal destruction time, fluence $F_S$ required to produce selective damage and $F_{NS}$, the fluence required to produce unselective tissue damage on the density factor for the targets.

## Detailed Description

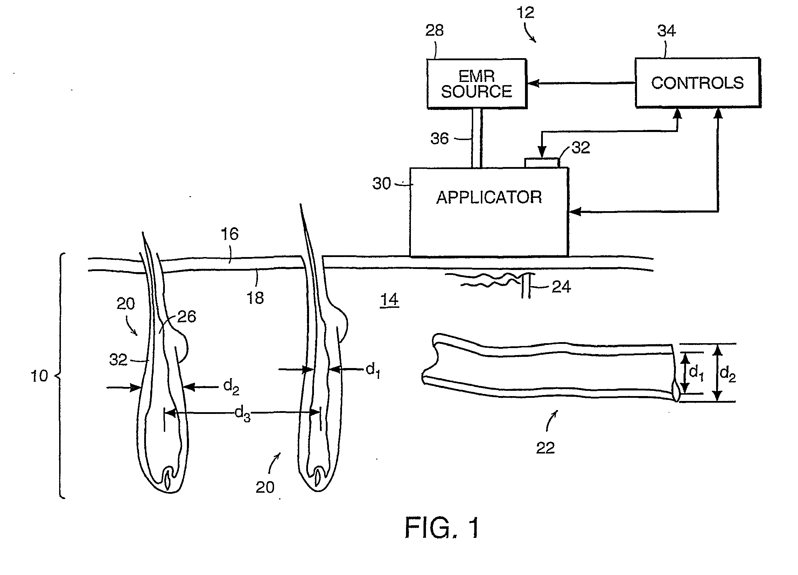

Fig. 1 is a schematic representation of a patient's skin 10 and of an exemplary system 12 for applying electromagnetic radiation (EMR) to the patient's skin for treating a variety of medical conditions. The skin 10 consists of a dermis 14 covered by an epidermis 16, there being a dermal epidermal (DE) junction 18 at the intersection of these two skin layers. Epidermis 16 is generally relatively thin, extending perhaps 30 to 200 microns from the skin surface, while the dermis 14 is thicker, extending on average approximately two to five mm from the DE junction. Pigmentation, which determines the color, and in particular the darkness, of a person's skin is contained primarily in epidermis 16 in the form of melanin which exists primarily on the epidermis side of the DE junction, but, particularly for darker skinned individuals (including tanned individuals), also exists throughout the epidermis. The darkness of a person's skin is frequently quantified on a Fitzpatrick scale, which ranges from I for very light skinned individuals to VI for very dark-skinned individuals.

There are many components within the skin on which EMR dermatological procedures may be performed. These include hair follicles 20, blood vessels 22, collagen 24 in the papillary dermis, etc. Hair follicles 20 may for example be irradiated to remove unwanted hair, for example facial or leg hair on women, and may in some cases also be irradiated to stimulate hair growth. The mechanism for stimulation can be soft reversible damage of the follicle matrix to stimulate blood delivery to the papilla. Treatment may also be performed to remove blood vessels 22 or other vascular lesions, which may include leg veins, spider veins, varicose veins or other blood vessels which either cause discomfort to the patient or which are consider unsightly and the removal of which is desired. Collagen 24 may be non-invasively remodeled, existing collagen being destroyed to facilitate regrowth of new collagen, for wrinkle removal and other purposes. The teachings of this invention may also be used for other dermatological treatments and for certain subdermal treatments, such as fat removal, acne (sebaceous gland) treatment or for other medical procedures normally performed by selectively applying EMR of an appropriate wavelength to a selected treatment area.

Apparatus or system 12 illustrates in schematic form the basic components of a system or apparatus for applying EMR radiation to a treatment area of a patient. Apparatus 12 includes a source of EMR 28, an applicator 30 through which the EMR is applied to the skin of the patient, a mechanism 32 which as shown operates in conjunction with applicator 30 to remove heat from the surface of the patient's skin, or in other words to cool at least the epidermis of the patient, and controls 34 which operate in accordance with the teachings of this invention to control, for example the power profile and/or duration of EMR from source 28 and/or to control applicator 30 to achieve a desired power profile, wavelength profile and/or duration for EMR applied thereto from source 28 and to control component 32 as required to achieve a desired cooling of the patient's skin.

EMR source 28 may be any of a variety of EMR sources currently or hereafter used or developed for EMR medical procedures, including various types of lasers, for example solid-state lasers and diode lasers, fiber lasers, flash lamps, filament lamps and other sources of incoherent optical radiation, a microwave source, an RF source, etc. When EMR source 28 produces radiation at one or more wavelengths, or wideband radiation, either of which includes wavelengths which are not desired for a particular treatment, source 28 and/or applicator 30 may include filters, wavelength slifters or other appropriate components to eliminate undesired wavelengths and/or to enhance desired wavelengths.

Further EMR source 28 may be a pulse source which remains on for a selected duration, may be a source which generates a sequence of pulses in rapid sequence, with short spaces between adjacent such pulses, such sequence of pulses being generated for a duration which is equal to the desired duration for the EMR signal, or may be a continuous wave (CW) signal, such CW signal being either continuously on or a sequence of short pulses of the type indicated above with small spaces between adjacent pulses. The power supply can also control the power profile of the EMR to obtain an optimum pulse shape which maximizes heating of the target and minimizes overheating of the epidermis. A CW source and/or the applicator 30 used therewith may be moved at a selected rate over a treatment area so as to provide a selected dwell time or pulse duration over each target area. Thus, when the terms "pulse duration" or "signal duration" are used herein, such terms should be interpreted to mean the total dwell time of any of the above EMR signals on a selected target area or portion thereof.

EMR from source 28 is applied through a suitable conduit 36 to applicator 30. For example, where source 28 is a source of optical radiation, conduit 36 may be an optical fiber or bundle of optical fibers. Alternatively, applicator 30 may be a radiation delivery head having EMR source 28 physically mounted therein. Other arrangements known in the art for delivering EMR radiation from a source to an applicator may also be utilized.

While applicator 30 is shown in Fig. 1 as being in contact with the patient's skin, and for most applications an applicator in contact with the patient's skin is preferred, this is not a limitation on the invention, and in some applications a non-contact applicator may be used, either with passive cooling, cryogenic spray cooling, etc., the specific applicator utilized varying with application. For example, where the applicator is also being utilized to cool or remove heat from the skin surface, good thermal contact is clearly preferred. Contact may also permit more efficient transfer of radiation into the patient's skin and may facilitate retro-reflection of radiation which, as a result of scattering in the skin, leaves the skin and is reflected back by the head toward the treatment area. Depending on the nature of the radiation applied, applicator 30 may be stationary over a treatment area for the duration of a given EMR pulse or signal or, where source 28 is a source of CW radiation, applicator 30 may move over the skin at a rate so that the dwell time of the radiation from the applicator over each portion of the treatment area is equal to the desired duration of the EMR signal. Applicator 30 may also determine or control the aperture size for the applied radiation.

Mechanism 32 may be any of a variety of components known in the art for cooling or removing heat from a patient's skin. For example, applicator 32 may be a waveguide, lens or plate of sapphire or other suitable material through which the radiation is applied to the patient's skin, the material of the waveguide/lens/plate having good heat transfer properties and being cooled by a thermoelectric device 32 being in contact therewith or by passing a cooling fluid, for example a cryogenic fluid, water, gas, etc., over the waveguide/lens/plate either periodically or continuously in manners known in the art. Applicator 30 could also be a block of a metal or other material having good heat transfer properties with open channels through which radiation is applied to the patient's skin, the block being cooled using one of the techniques previously indicated. For some embodiments, component 32 may be utilized to first heat the person's skin to the depth at which treatment is to occur and then used to cool the epidermis prior to irradiation. (See for example co-pending application Serial No. 09/078,055, filed May 13, 1998, the content of which is incorporated herein by reference, for a more detailed discussion on metal block applicators and the preheat/precooling feature). However, the teachings of this invention may eliminate the need for such precooling.

Controls 34 may be manually operated, but preferably include a microprocessor or other suitable processor programmed to control the operation of EMR source 28, applicator 30, and cooling component 32 in the manner to be hereinafter discussed to achieve a power profile and duration for the applied radiation which conform to the teachings of this invention. Controls 34 may receive selected inputs from source 28, applicator 30, temperature mechanism 32 and/or other components indicating such things as temperature of various system components and/or of the patient's skin.

As indicated earlier, there are at least two situations where conventional EMR dermatology treatments are not effective. These situations are (i) where the patient has dark skin, for example, patients with Fitzpatrick's skin types V and VI; and (ii) where the treatment area has non-uniform absorption characteristics such that a heater portion of the target area having high absorption at the applied EMR absorbs energy from the EMR and is heated thereby, the heater portion being spaced from portions of the target which absorb little or no EMR at the applied wavelength, but which are to be thermally destroyed or otherwise thermally treated. In the former situation, in accordance with the teachings of this invention, it has been discovered that when the EMR is applied at relatively low power over an extended time interval, heat produced as a result of EMR absorption by melanin in the epidermis has time to move to the skin surface and be removed, particularly if the skin surface is actively cooled, this parallel cooling of the epidermis during EMR irradiation of the target area permitting treatment to be effected without causing damage to the patients epidermis, even for patients having Fitzpatrick's V and VI skin. In the second case, in addition to the parallel cooling effect indicated above, the relatively long duration EMR signal also permits time for heat to diffuse from heater portions of the target area to the non-pigmented portions of the target to achieve the desired thermal destruction or other treatment of such non-pigmented portions. However, since the heater must remain intact to permit heat diffusion therefrom for the period required for heat to the desired temperature to diffuse to the non-absorbing target portions, the energy applied to the heater must, at least for the required pulse duration, be below the threshold at which the heater undergoes a phase change or transition which results in a significant loss of absorption at the EMR wavelengths, for example as a result of bleaching, melting, boiling, bubble formation or other destructive process on the heater. Thus, as discussed in far greater detail later, the invention involves the use of EMR signals of relatively low peak power delivered over a relatively long duration in order to achieve the objectives indicated above. Such low peak power, long duration EMR signals also permit use of lower cost EMR sources and, by permitting much lower power EMR sources to be utilized, are far safer to use, permitting in some instances further cost reductions by having treatments performed by less highly skilled and trained, and thus less expensive, individuals. Since the chromophore remains intact, even when a treatment is completed, multiple treatments may be performed during a single treatment session or within days of each other to permit safer, more effective treatment. The extended time interval of the treatment also facilitates feedback control.

While in the discussion to follow, the long duration EMR signals are sometimes referred to as "pulses," it should be understood, as indicated earlier, that long duration EMR signals in accordance with the teachings of this invention may be obtained in at least three different ways. First, the EMR signal may be applied as a single pulse of the desire duration. Such a pulse may for example be achieved by operating EMR source 28 for the prescribed duration, by using a shutter to pass radiation from the source to the target for such duration or by other techniques known in the art. When the EMR source pulses on and off at a relatively high rate, such source may be used as long as the maximum spacing between adjacent pulses is very short relative to the EMR duration and the average power of the pulse train for the duration of the EMR signal is sufficient to effect the desired treatment. The latter condition is necessary in order for requisite power and energy to be achieved without requiring a high peak power source. The third way in which the EMR signal duration may be achieved is to use a continuous wave (CW) source, which source may be a high repetition rate, high duty cycle source of the type indicated for the second option, and passing this source over the treatment area in for example the manner indicated in co-pending Application Serial No. 09/078,055 filed May 13, 1998, the signal duration in this case being the dwell time of the CW signal source over the target area. For this embodiment, the head delivering the EMR energy would be moved at a much slower rate then for embodiments where the teachings of this invention are not practiced.

Considering first the objective of achieving more effective parallel cooling so as to be able to more safely treat all patients, and in particular to be able to safely treat dark skinned (including tanned) patients, the problem with dark-skinned patients stems from the fact that for all patients there is some melanin concentration in the epidermis at the dermal/epidermal (DE) junction. The melanin concentration in this area increases for darker skinned individuals, and, as skin color darkens, melanin tends to increasingly appear throughout the epidermis. For very dark skinned patients, for example patients with skin types V and VI, there is significant melanin concentration at the DE junction, with lesser amounts of melanin throughout the epidermis. Thus, for treatments at a radiation wavelength which is preferentially absorbed by melanin, for example hair removal treatments where melanin in the hair shaft, hair matrix and other portions of the follicle is being targeted, significant heating of epidermal melanin can also occur, making it difficult to perform such treatments on dark skinned individuals. Thus, while heat from melanin absorption in the epidermis can migrate to the skin surface to be removed, either by air, by evaporating cryogen on the skin surface, by a cooled element in contact with the skin surface, or by other suitable means in as little as 0.1 milliseconds for melanin near the surface of the epidermis (melanin being near the surface at the epidermis for dark skinned individuals and/or for skin areas having an exceptionally thin epidermis), it typically takes at least 10 ms for heat from melanin at the DE junction to reach the skin surface, and generally takes significantly longer. It has been found that for optimal parallel cooling, parallel cooling being defined for purposes of this invention as skin cooling which occurs as a result of heat escaping from the skin's surface during irradiation, a radiation duration of at least 50 ms, and preferably 100 ms or more, is required.

In particular, while the time required for the front of the heat to flow from the DE junction to the skin surface may on average be approximately 10 ms, the epidermis also has a finite capacity for transferring heat per unit time. Thus, while some heat will be dissipated from the DE junction through the mechanism of parallel cooling with an irradiation duration of 10 ms, the epidermis does not have the capacity to remove most of the heat accumulated in the melanin at the DE junction during this time interval. It has been found that in order to hold down the temperature at the DE junction, low peak power irradiation for a duration of at least 100 ms is normally required, this duration being sufficient for the heat removal capacity of the epidermis to permit sufficient heat transfer to the skin surface so as to assure against overheating of the epidermis in general, and the DE junction in particular. Longer durations for the applied irradiation and/or more aggressive active cooling of the skin surface can further reduce or even eliminate heat rise in the epidermis, thereby enhancing both safety and patient comfort for EMR medical treatments in general and for optical dermatology treatments in particular.

With such long pulse durations, and with the lower peak power sources which may be utilized to deliver a desired energy to the target or treatment area with such long treatment times, it has been found that all types of skin, including skin types V and VI, may be treated safely without incurring thermal damage to the epidermis.

Further, the lower peak power required, particularly for pulse durations in excess of 100 ms, and in some cases in ranges up to several seconds, for example up to 20 seconds and more, permits optical sources having a peak power in the 100's of watts range, for example 100 to 200 watts, to be utilized, and possibly even less, permitting for example an 800 nm laser diode head having only one to three laser diode bars, as opposed to 10-100 bars for shorter pulse, to be utilized. Similar improvements can be obtained at other wavelengths. In some applications, this may even permit the EMR source to be a standard incandescent light bulb or other standard light source (for example, even the sun), appropriate filtering or wavelength shifting sometimes being required or desirable when such wide-spectrum sources are utilized. As is discussed in greater detail later, in some applications where low power sources are utilized, exposure times in the range of minutes, or in some cases hours, may be appropriate. The low peak power, long pulse duration treatment protocols discussed in this paragraphpermit optical dermatology procedures to be performed with smaller, lighter and significantly less expensive equipment, and also significantly simplifies thermal management problems, further reducing equipment size and cost.

Another significant advantage of utilizing the low peak power, long pulse technique of this invention is that its safer for the patient. In particular, while prior art systems can, if not properly utilized, cause damage to the patient's skin or, when used to treat for example wrinkles or hair on a patient's face, have the potential for causing eye damage or damage to other organs of the patient's body, the potential for harm to the patient as a result of improper use of the treatment apparatus is significantly reduced when low peak power optical sources are utilized. Potential injury to the operator is also significantly reduced. Further, the low peak power, long duration pulses allow sufficient time for the condition of the patient's skin or other treatment parameters to be monitored and utilized to control treatment parameters, for example EMR power and/or cooling, and to terminate treatment in the event a dangerous condition is detected before skin damage occurs. Such feedback control and protection are not practical with the short pulses currently utilized. Thus, while current techniques require that the treatment be performed either by a dermatologists or other physician, or at least by a highly skilled technician under the supervision of a physician, the low peak power apparatus which may be utilized in accordance with the teachings of this invention may permit hair removal, skin resurfacing and other dermatological procedures to be performed by less highly trained people, and possibly by cosmologists, barbers and the like. Home use may even be possible, particularly if power is reduced so as to provide only "hair management," for example functioning as a long term razor, rather then permanent removal. This, coupled with the potential for significantly lower cost equipment, will dramatically reduce the costs of such optical dermatology treatments, making them available to a far larger population.

In addition to the advantages discussed above which arise from the use of low power, long duration EMR signals for performing EMR dermatology, such signals are also useful where the target area has non-uniform absorption of radiation for the wavelengths of the applied EMR signal. Such non-uniform absorption, which is common for the human skin, means that within a given target area there are portions which are highly absorbent at the wavelength or wavelengths of the EMR source and there are portions in the target area which are either weakly absorbent of such radiation or totally non-absorbent. Where a dermatological treatment requires thermal destruction of such non-absorbing or wealdy absorbing portions, such result has not been possible with prior art high peak power, short duration EMR signals, frequently resulting in less than ideal results for various dermatological treatments including, but by no means limited to, hair removal, elimination of vascular lesions and skin resurfacing through collagen remodeling.

However, the low power, long duration signals of this invention are capable of effecting such treatments. More specifically, the procedure using a pulse duration $\tau$ < TRT becomes inapplicable when the target and the chromophore or heater are spatially separated or the absorption of the target is non uniform over its volume, and the treated part of the target has weak or no absorption, but another part of the target has significant absorption. If this is the case, the weakly absorbing part of the target has to be treated by the diffusion, radiation, convection or other transfer mechanism of heat from the strongly absorbing heater part. For example, for permanent hair removal, in accordance with current knowledge, it is necessary to damage all follicle structure up to the connective tissue sheath or the stem cells located in the bulge area at the outermost layer of the outer root sheath. Damage or destruction of follicle matrix and/or papilla are also desirable for hair removal. The heaters with high absorption of light are the melanin-containing hair shaft and matrix cells. But the targets to be damaged include stem cells and other follicular structures such as the papilla that do not have the required chromophores. These targets can be damaged by heat diffusion from the hair shaft or the matrix cells to the surrounding follicular structures that do not contain a useful chromophore. In order to accomplish this, the hair shaft or other chromophore must continue to absorb for the entire pulse duration and must not become thermally isolated from the remainder of the follicle which is to be damaged or destroyed.

Another example of spatially separated target and heater/chromophore is the treatment of telangiectasia, or leg veins. Permanent closure of the vessels probably requires coagulation of the vascular wall. In this case the heater is blood, due to the high absorption of hemoglobin and/or water. Coagulation of the wall requires heat diffusion from the blood into the wall.

When properly controlled, this treatment procedure can be used to safely deliver a clinically-effective thermal dose to a prescribed region surrounding the light-absorbing chromophores or heaters to cause a thermal lesion or a volume of denatured or coagulated tissue as required, while avoiding an absorption degrading change to the chromophore. This assumes that the chromophore used (e.g., water, melanin, hemoglobin) has a higher threshold for thermal damage than the surrounding tissue, and that the energy delivered is such that it does not cause the chromophore to explode or be otherwise be altered so as to lose or degrade its ability to absorb radiation.

The thermal dose is calculated according to formulas known to those skilled in the art, and is an Arrhenius integral of the applied temperature over the time of treatment. The boundaries demarcating treated tissue from untreated healthy tissue outside the treatment volume are sharp due to the Arrhenius nature of the thermal dose delivery, such that tissue outside the treatment volume receives less total thermal dose than that lying within the treatment volume. It should be noted that the treatment volume may extend beyond the region being irradiated, and depends on the properties of the irradiating source as well as the tissue properties. Additionally, cooling by external means (e.g. parallel cooling) or internal means (by thermal diffusion or blood perfusion) affect the final outcome of the treatment, and in particular heating outside the treatment volume or target area, for example epidermal and possible dermal tissue above the target area.

Thermal damage of such types of targets requires heat deposition of sufficient power in the heaters, and good heat transfer from the heaters to the targets. Heat deposition depends on the coefficient of absorption of the targets and the power density of the EMR within the tissue. Heat transfer depends on the distance between the target and the heater and the heat transmission coefficient of the tissue. However, at a sufficiently high temperature, both the absorption coefficient of the heater and the heat transmission coefficient from the heater to the other targeted tissues may be compromised by phase transition and destructive processes like bleaching, melting, boiling, and bubble formation for the heater, and possible for tissue adjacent the heater. To prevent these undesirable effects, the peak temperature of the heater has to be limited by a prescribed maximum value, $T_{1max}$, called hereafter the "heater collapse temperature." Simultaneously, to ensure the permanent damage of the whole target, the temperature should exceed some minimum prescribed value, "the thermal damage temperature," $T_2$, over the target volume. The latter temperature is smaller than the collapse temperature. The temperature within the tissue between the target and the heater should be below the boiling temperature of water to prevent thermal isolation of the target from the heater and other adverse effects. To meet the temperature limitations above, the power of the EMR should be controlled, and the pulse width has to be sufficiently long to deliver the energy needed. The thermal damage time (TDT) of the target is the time for the target temperature to exceed $T_2$ for a duration sufficient for irreversible target damage without damaging the surrounding tissue. TDT is thus the time to generate a temperature $T_2$ at the target by heat diffusion from the heater to the target. Because the temperature gradient is not sharp, part of the heat will leak from the target, resulting in the heated area being larger than the target. Nevertheless, TDT may be many times as long as TRT of the whole target while still achieving selective damage of the target with sparing of the surrounding tissue. The optimum pulsewidth $\tau_0$ of the EMR pulse should however be slightly shorter than or equal to TDT.

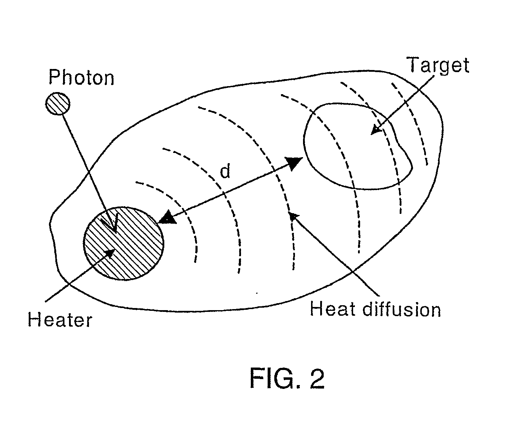

The mechanism for selective damage of the target by heat diffusion is illustrated in Fig. 2, this mechanism relating to therapeutic and medical treatments using electromagnetic radiation to selectively damage targets that are spatially separated from the light absorbing chromophore. In Fig.2, the distance between the heater and the target is $d$. Photons from an EMR source are absorbed by the heater. The EMR power density and pulse length are established so that the temperature of the heater $T_1$ should be below the thermal damage threshold $T_{1max}$ of the chromophore. Heat is transferred from the heater to the target by thermal diffusion or another mechanism such as by shock waves or steam.

Confined thermal damage of the target is achieved when the temperature of the target reaches $T_2$, but the external surrounding tissue remains below that temperature. This thermal damage temperature $T_2$ depends on the duration and the shape of the heating pulse. For proteins, $T_2$ is between 42-80°C, depending on the dwell time (i.e., determined by Arrhenius integral). It is usually not possible to damage the target without damaging the tissue between the target and the heater. After the end of the EMR pulse, the temperature of the target will continue to rise to a maximum some time later. The time delay between the end of the EMR pulse $\tau$ and moment of peak temperature of the target, that is TDT, is denoted by $\delta$ (i.e., $\tau$ = TDT-$\delta$). The pulse width should thus be equal to or shorter than the TDT, $\delta$ being significantly shorter than the TDT in most cases. Therefore, the pulsewidth for selective treatment may be considered to be equal TDT (i.e., $\tau$ = TDT).

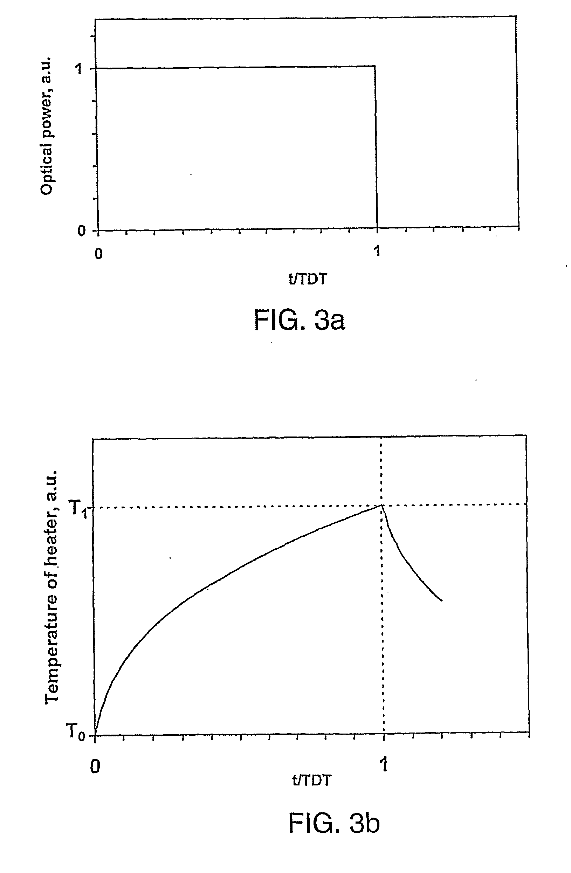

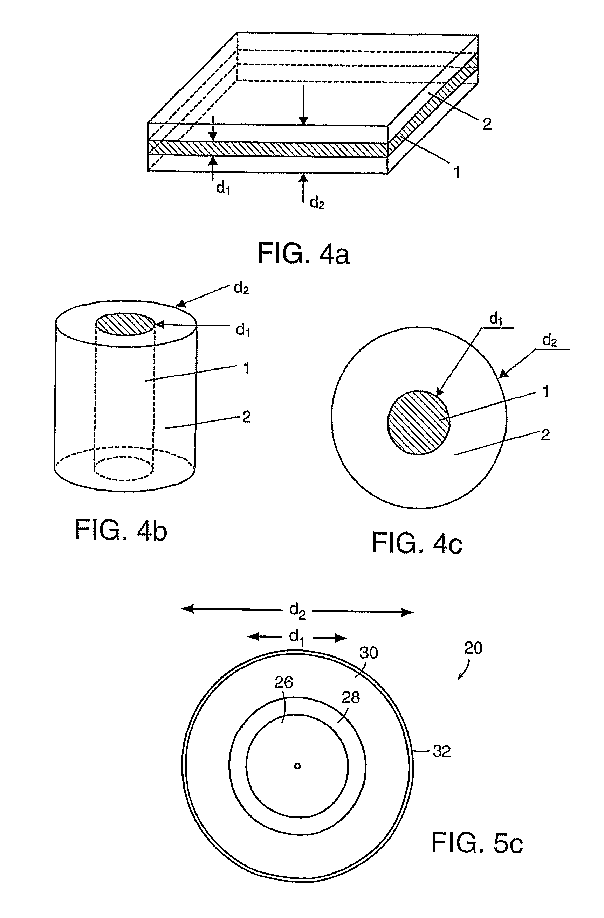

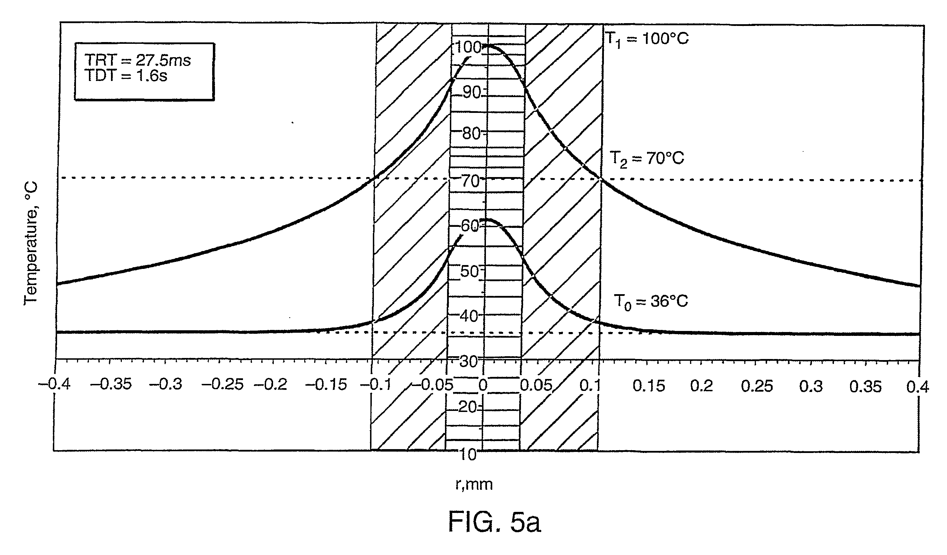

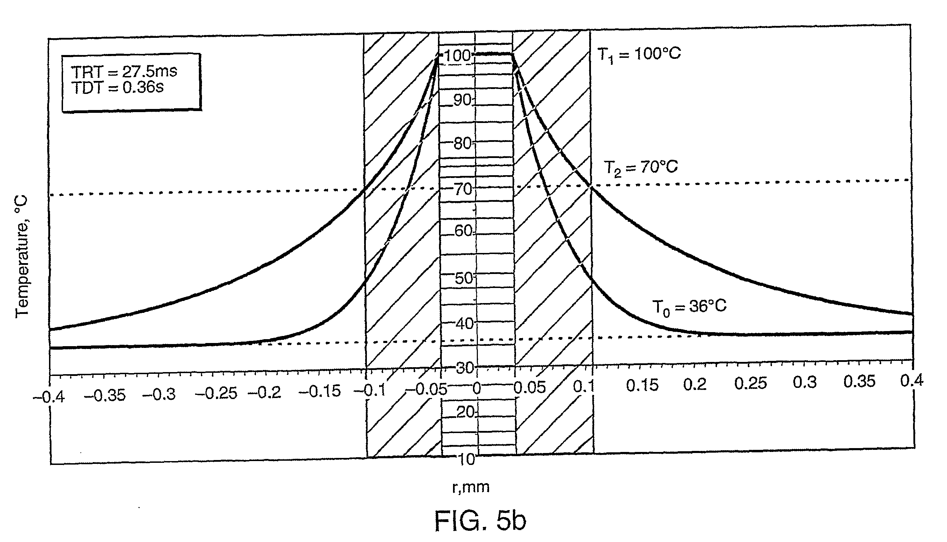

As a first example, a target that is a highly pigmented long cylinder with diameter $d_1$ surrounded by a treatment area with diameter $d_2$ (Fig. 4b) is considered, this model representing, for example, a hair follicle or a blood vessel. Two modes of heating are considered. The first mode is a "rectangular EMR pulse" (Fig. 5a), and the second mode is a "rectangular temperature pulse" (Fig. 5b). In the case of the rectangular EMR pulse, the temperature of the heater rises during the EMR pulse, and reaches $T_1$ at the end of the pulse (Fig. 3b). In the case of the rectangular temperature pulse, the temperature of the heater is constant during the EMR pulse (Fig. 3a). For both modes of heating, the temperature of the heater is below the collapse temperature $T_{1max}$, so absorbing properties of the of heater are not changed.

The sequence of thermal profiles during heating of the heater is depicted in Figs. 5a, 5b for the first mode and second mode of heating respectively, these profiles in the target and surround tissue being at different moments. The illustrative parameters for these profiles are $d_1$ = 70 $\mu$m, $d_2$ = 210 $\mu$m, collapse temperature $T_{1max}$ =100°C, corresponding to the boiling temperature of water, tissue damage temperature ($T_2$) = 70°C, associated with temperature of denaturation of proteins for pulsewidths in the range 10-1000 ms. The lower curve in both figures is the temperature profile at a time equal to the thermal relaxation time of the target (i.e. TRT=$d^2$/16k) where k is thermal diffusivity of the tissue; k=0.1mm$^2$/s, TRT=27.5 ms for the illustrative profile. At TRT, the temperature on the boundary of the target is significantly below the thermal damage temperature. The upper curve in both figures is the temperature profile at TDT when the target temperature reaches the damage temperature $T_2$. At this moment, the target is damaged, but the surrounding tissue is not. For the illustrative profiles, TDT=1600ms for the rectangular EMR pulse, and TDT=360ms for the rectangular temperature pulse. From Figs. 5a, 5b, it is seen that (a) the ratio TDT/TRT is 58 and 14 times respectively; thus, for both modes, pulse width $\tau$=TDT is significantly longer than TRT; and (b) at the end of TDT, the heated area is significantly larger than the damaged target. Thus, because of the spatial separation of highly pigmented areas and the target areas, the above technique differs from the classical case of selective photothermolysis in that the target is damaged not because of direct heating by absorption of EMR, but because of heat diffusion from the heater to the target. Fig. 5c is a cross-section through an illustrative hair shaft illustrating the relation of the profiles of Figs. 5a, 5b to the targeted hair follicle. In Fig. 5c, 26 is the hair shaft, 28 the inner root sheath, 30 the outer root sheath and 32 the location of stem cells (see also Fig. 1).

Heat diffusion strength depends on the geometry of the heater and the target. Three basic geometry examples, namely planes, cylinders and spheres (Fig. 4a-4c), are used to illustrate this. In all cases, a heater of size $d_1$ is assigned, located in the center of a target of size $d_2$. A geometrical factor value x= $d_2/d_1$ is also defined. As above, $T_1$ is the maximum temperature of the pigmented area, and $T_2$ is the damage temperature of the target ($T_1 > T_2$). The thermal damage time of the target is:

$$TDT = TRT \times r(x, \Delta)$$

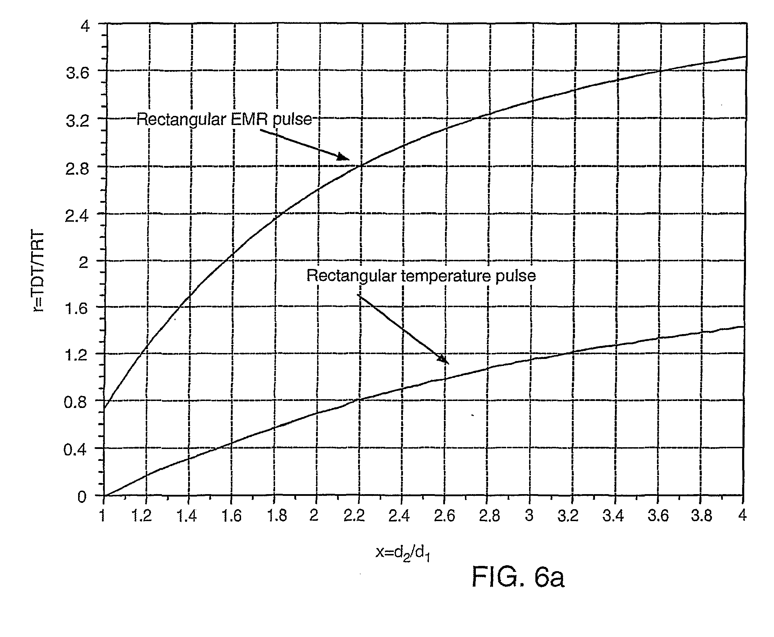

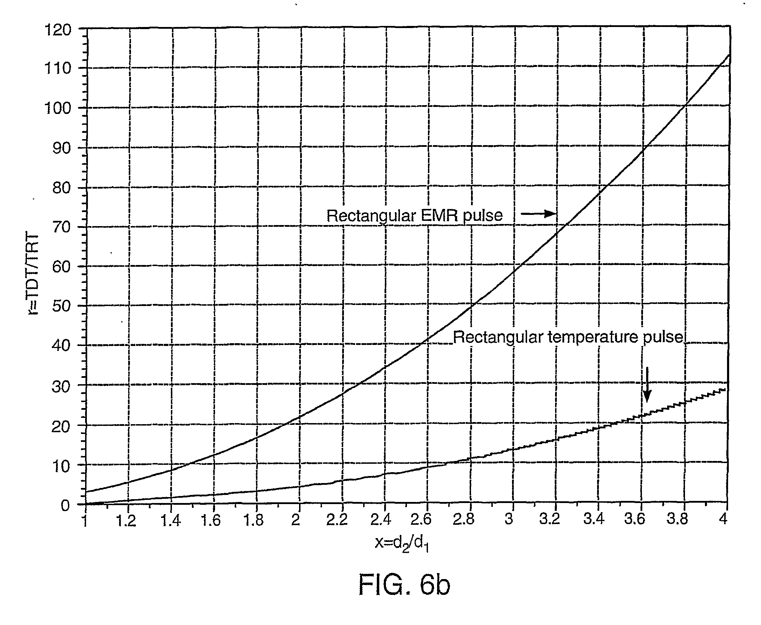

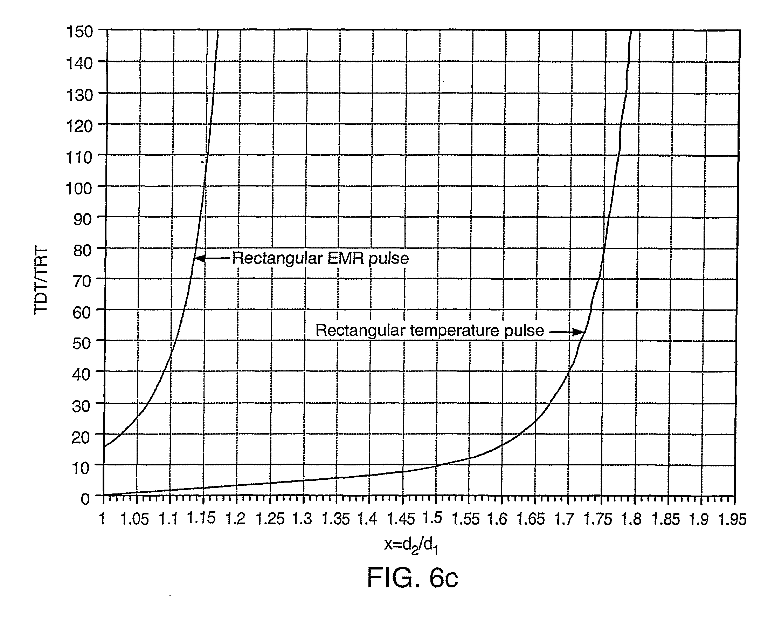

Here $r(x, \Delta)$ is a function of the geometrical factor and a temperature factor $\Delta$, where $\Delta = (T_2 - T_0)/(T_1 - T_0)$. $T_0$ is the temperature of the target and heater before irradiation. Normally $T_0$ is body temperature ($37°C$). Figures 6a-6c show the ratio $r(x, \Delta) = TDT/TRT$ as function of the geometrical factor x for two heating modes, the "rectangular EMR pulse" and the "rectangular temperature pulse", Fig. 6a being for a plane target (Fig. 4a), Fig. 6b for a cylindrical target (Fig. 4b) and Fig. 6c being for a spherical target (Fig. 4c). Parameters for the calculations were $T_1 = 100°C$, $T_2 = 70°C$ and $T_0 = 36.6°C$, resulting in $\Delta = 0.52$. Note that the ratio $r(x, \Delta)$ is not dependent on the size of the target or the thermal properties of the tissue. Several important conclusions follow from Figures 6a-6c:

1) The ratio TDT/TRT increases as the geometrical factor x is increased.

2) The value of this ratio is very different for plane, cylindrical and spherical targets. For plane targets, TDT is only a few times greater than the TRT, but for the same geometrical factor x, it is several dozen times greater than TDT for cylindrical and spherical targets.

3) For the same TDT/TRT, the size of the damaged area is smallest for a spherical target, next larger for a cylindrical target, and largest for a plane target. These results are to be expected because heat diffusion from plane, cylindrical and spherical targets is one, two and three dimensional respectively. The temperature profile is sharper and better localized for spherical heaters than for cylindrical heaters, and for cylindrical heaters it is sharper than for plane heaters. For the classical case of selective photothermolysis, the geometry of the target is not important because thermal damage takes place in the same area as the absorption of the EMR; however, it is important in the present case because, as a result of heat diffusion, thermal damage takes place in a different area than the absorption of EMR.

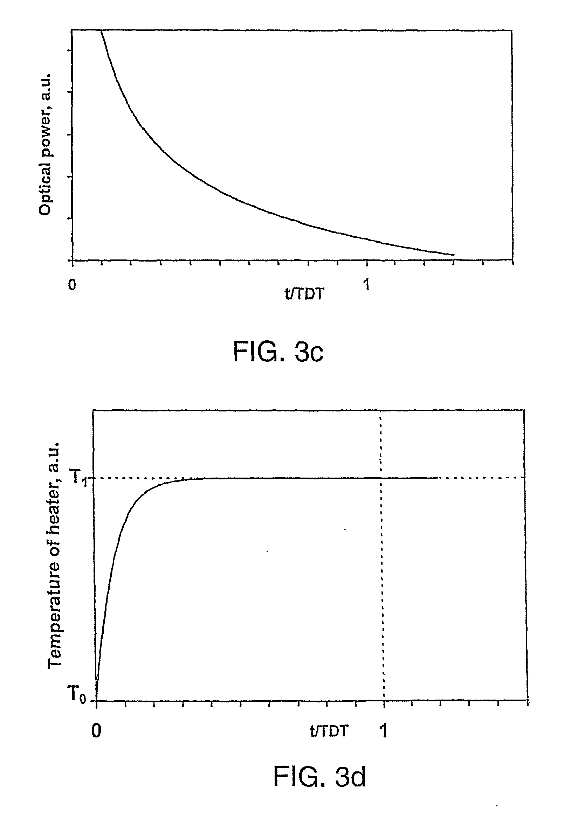

4) The ratio TDT/TRT depends highly on the heating mode. The rectangular EMR pulse mode (Fig. 3a, 4b) represents a more gradual heating mode because the temperature of the heater reaches the maximum $T_1$ at the end of the pulse (Fig. 4b). Ratio TDT/TRT is maximum for this mode. The rectangular temperature pulse mode (Fig. 3b, 4a) represents a more aggressive heating mode because the maximum temperature of the heater is found during the entire EMR pulse. The ratio TDT/TRT is minimum for rectangular temperature pulse mode. This mode requires a special shape for the EMR pulse with a high peak power at the beginning, and falling for the rest of the pulse (Fig. 3c) to maintain heat diffusion from the heater into surround tissue. Pulse power should be adjusted precisely to keep the temperature of the heater below its collapse temperature, $T_{1mex}$ (Fig. 3d). The power depends on the absorption coefficient of the heater, its size, and the attenuation of the EMR in the intervening tissue.

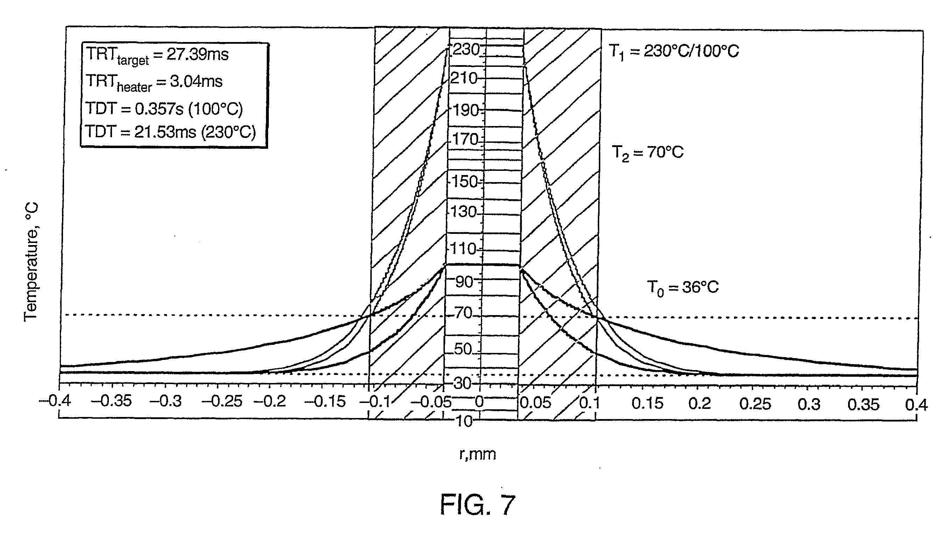

The ratio TDT/TRT also depends on the temperature factor $\Delta = (T_2 - T_0)/(T_1 - T_0)$. Fig. 7, which shows the influence of the maximum temperature of the heater $T_1$ for a cylindrical target, compares two cases having $T_1 = 100°C (\Delta = 0.53)$ and $T_1 = 200°C (\Delta = 0.20)$. TDT/TRT is close to one when the temperature of the heater is very high (for example 200°C). In biological tissue, such high temperatures can be expected with chromophores like carbon or melanin in the hair shaft.

Where the EMR pulse is square, with its amplitude and duration chosen to heat the heater just below $T_1$ and the target to $T_2$, the three basic geometries of the target are used, the part with high absorption is located at the center of the target, $d_1$ is the size of the heater and $d_2$ is the total size of the target, and it is assumed that all tissue of the target surrounding the heater should be damaged by heating to above $T_2$, TDT of the targets and input power density P and fluence F for rectangular EMR pulse follows from thermal diffusion theory and are given by formulas in Table 1:

Table 1

| N | Quantity | Notation [Dimensionality] | Approximate expression for a particular target geometry; | | |
|---|---|---|---|---|---|
| | | | Planar | Cylindrical | Spherical |
| 1 | Thermal relaxation time of the heater | $\tau_r$ [s] | $\tau_r = \dfrac{d_1^{\,2}}{8 \cdot k}$ | $\tau_r = \dfrac{d_1^{\,2}}{16 \cdot k}$ | $\tau_r = \dfrac{d_1^{\,2}}{24 \cdot k}$ |
| 2 | Thermal relaxation time of the target | TRT [s] | $TRT = \dfrac{d_2^{\,2}}{8 \cdot k} = $ $= x^2 \cdot \tau_r$ | $TRT = \dfrac{d_2^{\,2}}{16 \cdot k} = $ $= x^2 \cdot \tau_r$ | $TRT = \dfrac{d_2^{\,2}}{24 \cdot k} = $ $= x^2 \cdot \tau_r$ |
| 3 | Thermal damage time | TDT [s] | $TDT = \dfrac{TRT}{2 \cdot x^2} \cdot$ $\left[ \left( \dfrac{D-\Delta}{1-\Delta} \right)^2 - 1 \right],$ $D = \exp(-x^2) + $ $1.8 \cdot x \cdot erf(x)$ | $TDT = \dfrac{TRT}{x^2} \cdot$ $\cdot \exp\left( \dfrac{D - 0.3 \cdot \Delta}{1-\Delta} \right),$ $D = 0.6 + $ $2 \cdot In(x) - $ $Ei(-1.4 \cdot x^2)$ | $TDT = $ $\begin{cases} 0.9 \cdot \dfrac{TRT}{x_t^2} \left[ \left( \dfrac{1-\Delta}{D-\Delta} \right)^2 - 1 \right] \\ D - \Delta > 0 \\ \infty, D - \Delta \leq 0. \end{cases}$ $D = 0.7 \cdot \dfrac{erf(1.3 \cdot x)}{x}$ |
| 4 | Input power density | P [W/cm²] | $P = \dfrac{p \cdot c}{\mu_a q} \cdot \dfrac{1.1 \cdot x^2}{TRT} \cdot$ $\dfrac{T_1 - T_0}{\sqrt{1 + 2.1 \cdot x^2 \cdot \dfrac{TDT}{TRT}} - 1}$ | $P = \dfrac{p \cdot c}{\mu_a q} \cdot \dfrac{x^2}{TRT} \cdot$ $\dfrac{T_1 - T_0}{\ln\left( 1 + 1.4 \cdot x^2 \cdot \dfrac{TDT}{TRT} \right)}$ | $P = \dfrac{p \cdot c}{\mu q} \cdot \dfrac{0.3 \cdot x^2}{TRT} \cdot$ $\dfrac{T_1 - T_0}{1 - \dfrac{1}{\sqrt{1 + 1.2 \cdot x^2 \cdot \dfrac{TDT}{TRT}}}}$ |
| 5 | Input fluence | F J/cm² | F = P·TDT | F = P·TDT | F = P·TDT |

The notations and the basic parameters of the problem are explained in Table 2:

Table 2

| Variable | Dimensionality | Name | Assumptions and relations |
|---|---|---|---|
| K | $cm^2\ s^{-1}$ | Thermal diffusivity | Assumed to be the same all over the target |
| P | $G\ cm^{-3}$ | Density | Assumed to be the same all over the target |
| C | $J/(g^oK)$ | Specific heat | Assumed to be the same all over the target |
| $\mu_a$ | $cm^{-1}$ | Tissue absorption coefficient | Assumed to be zero outside the heater |
| Q | a.u. | The ratio of radiance to the input power density | |
| $d_1$ | Cm | Thickness or diameter of the heater | |
| $d_2$ | Cm | Thickness or diameter of the target | $d_2 > d_1$ |
| $d_3$ | Cm | Mean spacing between of the target | $d_3 > d_2$ |
| $T_0$ | ˚C | Initial temperature of both the target and the surrounding tissue | $T_0 = 37˚C$ |
| $T_{1max}$ | ˚C | Temperature of heater absorption loss, collapse temperature | $T_{1max} = 100˚\ C\text{-}\ 250˚\ C$ |
| $T_1$ | ˚C | Maximum temperature of the heater (absorber) | $T_2 < T_1 \le T_{1max}$ |
| $T_2$ | ˚C | Temperature of irreversible damage of the tissue | $T_2 = 70˚\ C$ |
| Δ | a.u. | Temperature factor, temperature ratio | $\Delta \cong \dfrac{T_2 - T_0}{T_1 - T_0} < 1$ |
| X | a.u. | Geometrical factor, diameter ratio | $x \cong d_2 / d_1 > 1$ |

Fig. 6a-6c shows dependence of TDT on x. The conditions for the calculation of this figure are: $T_1=100˚C$ (boiling temperature of water), $T_2=70˚C$ (temperature of denaturation of protein for dwell time 0.1-1s) and x=1 to 4.

The value of the TDT can be up to 4 times longer than the TRT for plane targets, 120 times for cylindrical, and 500 times for spherical targets. Typical parameters of treatment for hair follicle and spider veins given by the formulas are provided in tables 3 and 4.

Table 3. Optimum pulsewidth for hair-follicle treatment

| Hair type/ diameter | TRT of hair shaft, ms | TRT of hair follicle, ms | Halting hair shaft growth | | | Permanent hair-follicle destruction | |
|---|---|---|---|---|---|---|---|
| | | | TRT of hair matrix, ms | TDT of papilla blood vessel, ms | | TDT of stem cell, ms | |
| | | | | Rectangular temperature pulse | Rectangular light pulse | Rectangular temperature pulse | Rectangular light pulse |
| Fine/30$\mu$ | 0.6 | 5.4 | <0.3 | 0.5 | 1.5 | 30 | 115 |
| Medium coarse/70 | 3 | 27 | <2 | 2.7 | 8.5 | 170 | 610 |
| Large coarse/ 120$\mu$ | 9.6 | 87 | <5 | 8 | 21 | 510 | 1800 |

Table 4. Optimum pulsewidth for treatment of spider veins

| Diameter of vein, mm | Wall thickness, mm | TRT of blood volume, ms | TRT of vein, ms | TDT of vein and fluence F | | | | | | | |
|---|---|---|---|---|---|---|---|---|---|---|---|
| | | | | λ=577nm | | | | λ=800 or 1060nm | | | |
| | | | | Rectangular temperature pulse | | Rectangular light pulse | | Rectangular temperature pulse | | Rectangular light pulse | |
| | | | | TDT, ms | F, J/cm$^2$ | TDT, ms | F, J/cm$^2$ | TDT, ms | F, J/cm$^2$ | TDT, ms | F, J/cm$^2$ |
| 0.1 | 0.035 | 0.5 | 5 | 40 | 10 | 130 | 20 | 40 | 590 | 140 | 1090 |
| 0.25 | 0.08 | 4 | 35 | 150 | 6 | 515 | 15 | 150 | 335 | 610 | 615 |
| 0.5 | 0.12 | 335 | 135 | 215 | 4 | 740 | 6 | 215 | 115 | 1200 | 200 |
| 1 | 0.15 | 260 | 540 | 240 | 4 | 670 | 5 | 240 | 50 | 2400 | 90 |

EP 2 316 372 A1

Treatments for which the teachings of this invention are particularly adapted include wrinkle, hair and vascular lesion removal.

For wrinkle removal, since collagen provides the dermis with its basic structural integrity, it is generally believed that the removal of wrinkles in human skin can be accomplished by restructuring the dermal collagen. It is further understood that the process of collagen destruction, as well as collagen production, can be mediated by heating the collagen-containing portions of the dermis. This process of collagen destruction and collagen formation takes place over a rather broad temperature range (i.e., roughly 45-70 degrees Celsius). Increased collagen formation can also occur as a result of heating collagen/fibrocytes within the papillary dermis to a sublethal temperature above normal body temperature, this effecting/increasing fibrocyte metabolism. Furthermore, it is desirable that this lethal or sublethal heating take place primarily in the papillary dermis, the portion of the dennis closest to the dermal-epidermal (D-E) junction. Accordingly, this treatment is targeted at the upper portion of the dermis, from approximately 100 microns to approximately 1mm below the upper surface of the epidermis, and may be particularly targeted to the upper portion of this region. It should be noted that the process of wrinlde removal described above does not require the removal of the epidermis, usually referred to as "skin resurfacing", and is therefore less traumatic and more desirable as a cosmetic procedure. However, even in skin resurfacing, collagen restructuring is thought to be the fundamental process leading to a desirable wrinkle free result.

To heat the papillary dennis to the required temperature range, the melanin layer located at the D-E junction may serve as the chromophore for light absorption in the wavelength range approximately 400-1500 nm. While melanin is normally optimally absorbed in a wavelength band from approximately 500 nm to 1300 nm; this wavelength range is extended due to the added skin protection afforded the epidermis by the extra long pulse.

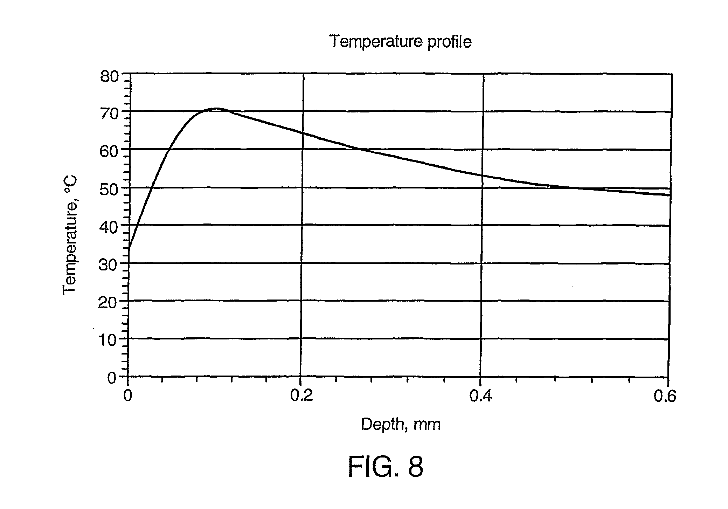

Using the discussion above as to the utility of using light pulses having long durations, preferably greater than 100 ms, to obtain maximum protection of the epidermis through parallel cooling, this wrinkle removal technique preferably requires pulse durations from 100 ms to 1 second or more. Depending on the skin type and other factors, times outside these ranges might also be employed. Through a combination of long pulse illumination of the dermis and contact cooling, an optimum temperature profile is produced in the papillary dermis for the restructuring of collagen, and accordingly wrinkle removal. Figure 8 shows the calculated temperature distribution for a cooled surface (10 degrees Celsius) and a laser pulse duration of one second at roughly 150 J/cm$^2$. With this curve, collagen remodeling extends about 200 microns into the capillary dermis, which should be sufficient for wrinkle removal. The target region of the papillary dermis mentioned above is shown to be in the range of 50-70 degrees Celsius.

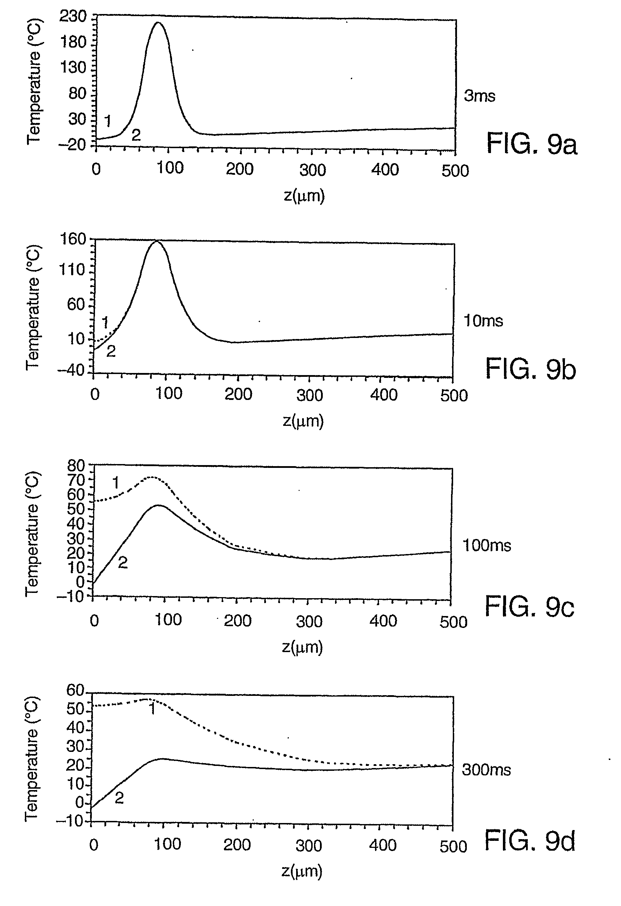

An explanation as to why the peak of the heating curve flattens out in the dermis in both Figs. 8 and 9 with increased pulse duration is as follows: Without cooling, a temperature distribution having its peak at the D-E junction (approx. 100 microns deep) is established. This distribution extends upward toward the skin surface and downward into the dermis. With parallel cooling, heat is preferentially transferred to the skin surface, depressing the portion of the heat distribution curve closest to the surface. Since the portion of the curve toward the dermis is not controlled as strongly by the surface cooling, if at all, and is largely mediated by the vasculature in the dermis, the portion of the heating curve in the dermis is not as changed (reduced) as is the portion closest to the surface of the skin. Fig. 9 illustrates this effect reasonably well at lower power (i.e., 50 J/cm$^2$) which may be suitable for some applications such as hair removal. Fig. 8 illustrates the effect using three times the fluence, a regimen more suitable for wrinlde removal. This treatment may be concentrated in areas containing substantial wrinkles to further protect against skin damage.

An alternative technique for utilizing the teachings of this invention for wrinkle removal is to utilize a source operating in a wavelength band preferentially absorbed by water, for example a range of 0.95 micrometers to 1.9 micrometers and 2.1 to 2.4 micrometers, and otherwise irradiating and cooling as indicated above. Radiation in the wavelength band indicated, while preferentially absorbed by water, are not so strongly absorbed that they cannot migrate at least several millimeters into the papillary dermis, heating water in the tissues of the papillary dermis sufficiently to raise the temperature of collagen in this area, resulting as indicated previously in the restructuring of such collagen.

There are a number of ways in which the teachings of this invention may be utilized for hair removal. In particular, in practicing the teachings of this invention, melanin in the hair shaft may function as a chromophore as well as melanin in the lower portions of the follicle near/in the matrix and near the papilla. Each hair follicle has stem cells 32 which are, as shown in Figs. 1 and 5c, at the outer side of the outer root sheath 30 in an area at a depth of approximately 0.5 to 1.5 mm from the skin surface. This is sometimes referred to as the bulge area of the follicle. Since the stems cells in general do not contain a chromophore, these stem cells can usually be heated and destroyed only by heating chromophores adjacent thereto, and permitting heat from such chromophores to be transferred to the stem cells. The most convenient naturally-occurring chromophore adjacent to stem cells is the melanin in the hair shaft 26 itself. To destroy the stem cells, the hair shaft must be irradiated for a sufficient period of time for heat from the hair shaft to be transferred to the stem cells. For permanent hair-follicle damage, in accordance with current knowledge, it is necessary to damage stem cells that are located in the bulge area at the interface of the outer root sheath and the connective tissue sheath. One can also irreversibly damage a hair follicle at the level of the dermis by replacing it with connective tissue and blocked growth of new hair bulb. It is important that the hair shaft or other heater being utilized not be destroyed during this entire period (i.e., that the hair shaft or other heater - i.e., melanin in the matrix - does not become so hot that it becomes denatured and ceases to function as a chromophore). However, charring or carbonizing of the hair shaft or other heater is acceptable since this does not reduce absorption, and may in fact increase absorption. Further, since water vapor can interfere with the transfer of heat from the hair shaft or other heater to the stem cells or other target, it is desirable that the temperature of the tissue be kept below approximately 100-110˚C, the temperature at which formation of water vapor may occur. Thus, the peak power of the optical radiation applied to the hair shaft/heater for this application should be low enough so that the hair shaft/heater does not heat to above approximately 100˚C by the end of the treatment, and the heating should last for a time sufficient for the heat from the hair shaft/heater to be transferred to the outer sheath of the follicle where the stem cells are located and/or other appropriate target. As is discussed later, this time will vary somewhat with the size of the hair shaft and follicle. Where the applied radiation is at a wavelength selectively absorbed by fat, the sebaceous gland, which primarily contains fat or lipid, and which is also located at the depth of the bulge, may also function as a chromophore for the stem cells, either in addition to or instead of other chromophores discussed herein. With proper focusing, it may also be possible using wavelengths previously discussed to target water in the stem cells and/or tissue surrounding the stem cells and/or in/surrounding other appropriate target.

While the melanin in the hair shaft (and possibly in the stem cells for type IV patients), the lipid in the sebaceous gland and water in tissue surrounding the hair shaft (and possibly in the stem cells) are the only naturally-occurring chromophores adjacent to or in stem cells, it is also possible to introduce an artificial chromophore into this region for purposes of destroying the stem cells. Thus, a dye could be applied to the hair shaft, which dye migrates down the hair shaft at least to the level of the bulge, or an artificial chromophore such as carbon particles or magnetic particles of a selected optical quality can be applied to the skin and either naturally migrate into the follicle region or be forced into the follicle region using various techniques known in the art. It is also possible to epilate the hair shaft before applying the chromophore to facilitate its migration into the follicle region. With any of these techniques, the optical source would still be a relatively low peak power source of a wavelength appropriate for the chromophores being utilized, and would be applied for at least a sufficient duration so as to permit the heating of the chromophores to a temperature sufficient to damage or destroy the stem cells and/or other appropriate target, and for the heat from the chromophores to be transferred to the stem cells. Preferably, the chromophores are heated to temperature to prevent chromophore destruction and ablation (for example 100˚C-300˚C depending on the chromophore), or slightly above, so that with thermal losses as the heat migrates to the stem cells, the stem cells will still be heated to approximately 65˚C-70˚C, as required for their destruction. It is also preferable that temperatures above 65˚-70˚C not reach much beyond the stem cells so as to avoid pronounced dermal damage. This is generally not a problem for regions of low density hair, but can be a problem for high density hair regions, where there can be accumulation of heat from adjacent follicles in dermal regions therebetween.

More specifically, since heat flux will leak out of the target, the heating area will be larger than the target. This increases the risk of overheating the tissue surrounding the target and thus the risk of nonselective damage. Very roughly, the fluence to produce nonselective bulk tissue damage $F_{NS}$ is $(d_3/d_2)^n$ times greater than the fluence required to produce selective damage $F_S$, where $d_2$ and $d_3$ are the target size and distance between centers of the targets, and n is 1, 2 or 3 for planar, cylindrical and spherical targets, respectively. As a first approximation, $F_{NS}/F_S$ is proportional to the ratio of target volume and tissue bulk volume and independent of pulsewidth, the risk of nonselective damage increasing in the following order: spherical, cylindrical and planar targets.

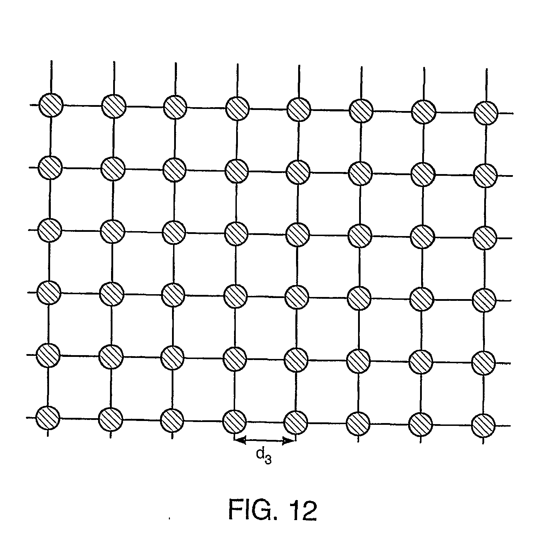

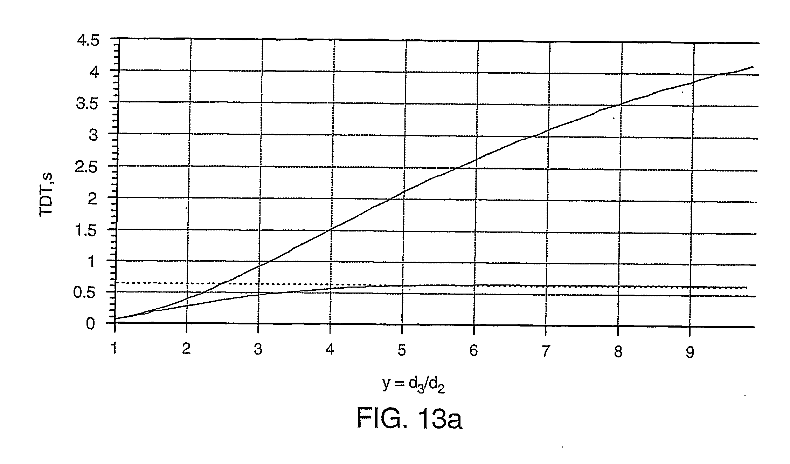

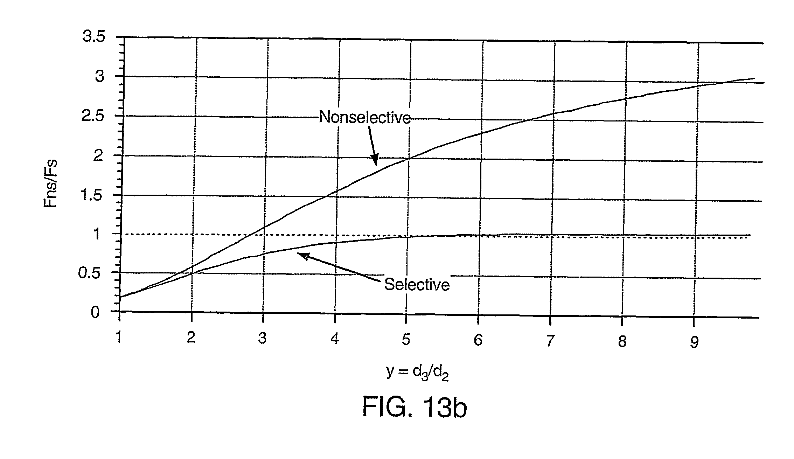

More precisely, for the ideal model of cylindrical targets with equal spacing (Fig. 12), Figs. 13a, 13b show the dependence of TDT, $F_S$ and $F_{NS}$ on density factor $y = d_3/d_2$ for a rectangular temperature pulse. As seen from Figs. 13a, 13b, TDT and $F_S$ decrease in y beginning with y = 5. This effect is explained by the influence of heat fluxes from neighboring targets. But at the same time, the ratio $F_{NS}/F_S$ starts dropping at y = 5. So for y < 5 the range of safe fluences is going to be very narrow and the risk of nonselective damage will increase.

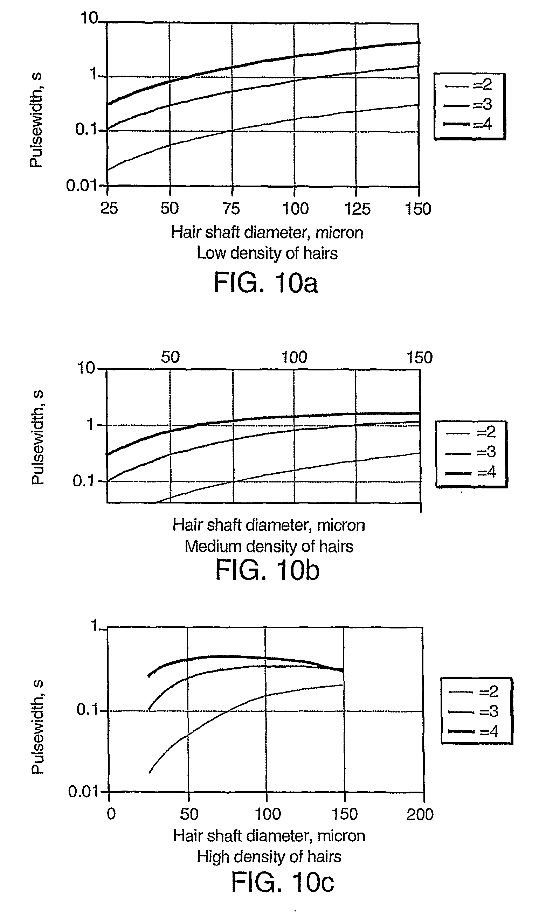

More specifically, hair follicle heating to achieve permanent hair removal is usually accomplished through light absorption by hair shaft melanin, melanin near the follicle matrix and/or artificial dye. This dye could be applied to the hair shaft and/or follicle or in the space created by epilation of the hair shaft. The feature of the proposed method is that melanin or artificial dye heating continues until a certain thermal dose or temperature is reached and lasts as long as necessary for a protein denaturation zone (or thermal lesion) to extend to the outer sheath border where stem cells are located and/or to another appropriate target. The thermal history and spatial profile of the temperature are primarily defined by two conditions. First, light absorption by the melanin or artificial dye, which should not decrease appreciably with heating. This means that there should be no drastic change such as melting or evaporation of the hair shaft or artificial dye, or photo-bleaching of the melanin or artificial dye, since this would cause follicle heating to stop or become ineffective. Second, the hair shaft or artificial dye and outer root sheath must not be heat-insulated from each other by, for example water vapor created by boiling of tissue water. The first condition is met if hair shaft temperature is not over one of the following: (a) 220-250˚C, (b) a temperature of artificial dye photo-bleaching, (c) the temperature of dye evaporation. This latter temperature depends on the dye type. The second condition is satisfied if the hair shaft or artificial dye temperature does not exceed the tissue boiling temperature, which is about 100-110˚C. The method thus requires that the temperature of the chromophores (melanin or artificial dye) be low enough to prevent its bleaching during light heating of a hair follicle. This temperature is preferably below the water boiling temperature (i.e., Tc or $T_{max}$=100-110˚C). To maintain the temperature of the chromophore at or below $T_{max}$, it is necessary to transport heat from the chromophore to the surrounding tissues (inner root sheath and outer root sheath) while adding heat to the chromophore. The power needed to ensure the heat production above, P(t), depends on the diameter of the hair shaft. The formula for determining P(t) appears in Table 1 for a rectangular pulse: The dependence of $\tau_0$ on the diameter of the hair shaft is demonstrated in Figs 10a-10c. For areas having widely spaced hair, the dependence is described by formula (3), with $\tau_0$ being approximately in the range 15ms - 5s (Fig. 10a). In the case of dense hair, because of the cumulative heat production by the hairs, $\tau_0$ is less than 400ms (see Fig 10c). The above procedure of hair shaft heating ensures the total destruction of stem cells independent of the stage of hair growth. If hair is in the anagen stage and melanin is used for absorption, then stem cell damage is accompanied by matrix cell damage, since the melanin concentration and its absorption coefficient in the matrix is higher than that in the hair shaft. In this case, the matrix temperature exceeds $T_{max}$. Through higher pulse duration, the structures of hair follicles thus become damaged. All these factors lead to a disruption of hair growth, and increased potential for permanent damage of the hair follicle, and thus permanent hair removal.

Another important effect is in the breakdown of the mechanical bond between the hair shaft and the dermis when thermal damage of the outer root sheath cell occurs. Owing to hair shaft temperature limitation during a treatment, the hair shaft remains intact and could be easily removed. Thus, the maintained hair shaft provides an objective criterion for hair follicle destruction; this criterion consists of mechanical removal of the hair shaft by gently pulling it out. The hair shaft can be removed with all or part of the follicle structure, including stem cells. This can be an additional mechanism of permanent hair removal. The hair shaft can be welded to the inner root sheath either with or without support of biological solder injected in the gap between hair shaft and inner root sheath.

A procedure for defining the parameters of the light treatment for this method might thus be:

1. Finding diameter $d$ and absorption coefficient $\mu_a$ for a typical hair shaft. Both $d$ and $\mu_a$ can be found by the use of standard methods. For example some hair (for statistical purposes) are mechanically removed and a diameter $d$ is measured by means of a divider, and absorption $\mu_a$ at the wavelength (wavelengths) is measured by use of a spectrometer. The above wavelengths are those of the light source applied for hair removal. Both parameters can be defined by use of a contact microscope equipped with CCD-camera. If an artificial dye is used, $\mu_a$ is defined based on the concentration thereof.

2. Setting $T_{1max}$ and following formulas in Table 1 to calculate the input flux.

3. Defining optimum pulse duration according to formula (3). For dense hair, a duration no more than 600 ms is chosen according to Fig. 10c.

4. Choosing type of skin according to standard methods, for example by measurement of reflection coefficient of skin for different wavelengths.

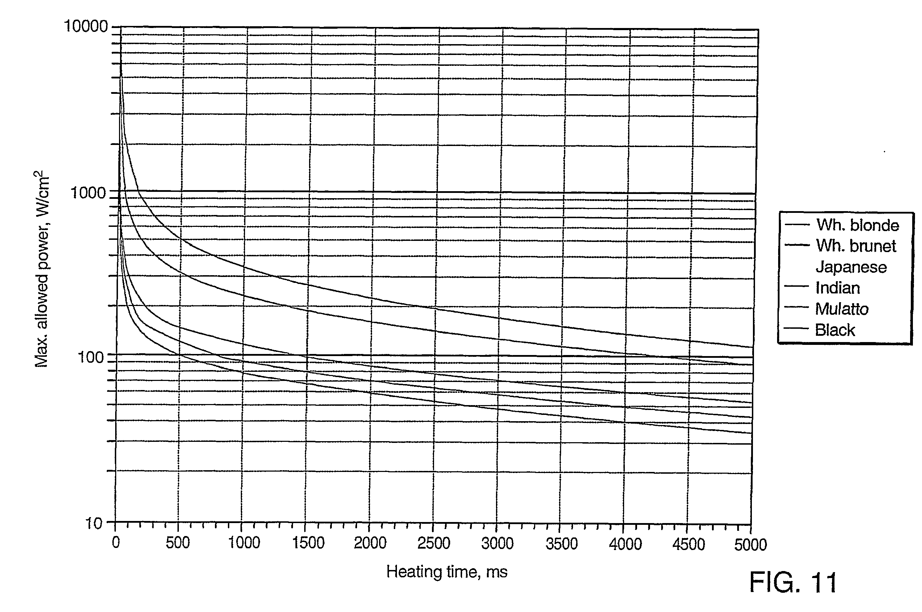

5. Using the skin type and the pulse duration for the selected wavelength to find the accepted maximum flux and fluence (see Fig. 11).

6. Choosing the flux for treatment as the smallest value of (a) the flux defined on condition of keeping epidermis intact (see Fig. 9) and (b) the flux providing hair heating to temperature $T_{1max}$ (see Eq. 1).

The above procedure may be carried out by applying the optical radiation locally or in a pattern with or without parallel cooling to achieve a spatial and temporal temperature profile and thermal dose map, which may include using a compound sequence of interrupted pulsatile irradiation, or a quasi-continuous irradiation scheme, whereby the position of the light source is scanned or patterned over the area to be treated.

An alternative procedure would be to choose a test sample before treatment. While hair shaving is not done, mechanical cutting is possible so long as the remaining length of the hair is sufficient to remove it.

Once minimum flux and pulse duration are chosen and the treatment is performed on the test sample, one can check if it is possible to pull the hair out with minimal force and that there has been no epidermal damage. If the hair does not come out and the epidermis stays intact, the pulse duration is increased by, for example 50-100ms, and the steps are performed again, ranging up to a maximum pulse duration of about 5s. Then the flux is augmented and the treatment procedure is duplicated with a rise of the flux from minimum to maximum. If certain values of the flux and pulse duration result in the hair coming out under gentle pulling while the epidermis stays intact after the treatment, then these parameters are suitable operational parameters for hair removal. If a certain value of flux and pulse duration result in damage of the epidermis but the hair pulls out with a great effort, then it may not be possible to remove hair from the skin of this patient by use of the invention. Experimental results achieved strongly suggest that it should be possible to treat all patients utilizing the teachings of this invention.

While for an individual hair shaft and hair follicle, the most appropriate set of exposure parameters can be chosen, as indicated above, the heterogenicity of hairs and hair follicles for a patient have to be considered in designing the most appropriate treatment of that patient. Different hair and hair follicles with similar properties can be grouped together. The more specific the differentiation of the group, the more appropriately each group can be treated. In general, division into a few group will allow treatment close to optimal treatment parameters. By applying multiple EMR pulses to the same area, each pulse's parameters optimized for a certain subset of hair follicles, allows more complete hair removal. These multiple pulses could either be applied in a short time interval over a single treatment or delivered during different treatment sessions. This technique effectively thins the hairs in the area, eliminating for example thinner hair first, thereby mitigating interaction problems in dense hair areas. This is because destruction of thinner hairs can occur before the TRT for the thicker hairs is reached.

A special mode of treatment is the delivery of multiple pulses with increasing flux from pulse to pulse. In this treatment mode, the thermal destruction of certain chromophores is intentional and can be described as "optical fuse". The calculations above demonstrate that with increasing flux, the threshold for hair shaft ablation is shifted to hairs containing less chromophore. If the outer root sheath cells are already destroyed by the preceding pulse, than the following pulse with a flux above the threshold for hair shaft ablation can remove the chromophore that is not needed anymore because this particular hair follicle is already destroyed. By removing the chromophore with increased flux, the EMR will only be absorbed by the remaining hair shafts with less chromophore. These hair follicles need a higher flux to generate sufficient temperature rise within the hair follicle. The maximum flux of this train of pulses is limited by the flux that is tolerated by the epidermis. This strategy of delivering a train of pulses, each targeting a different subset of hair follicles, and reduction in absorption of unnecessary EMR by removal of chromophores within already destroyed hair follicle is a novel treatment strategy. A higher ratio of damaged hair follicles within a treatment area can be expected with less danger of thermal damage, even for relatively dense hair areas. This is substantially different from a train of pulses each with the same flux and pulse duration, targeting the same subset of hair follicles. However, using parameters of EMR what are below the threshold of chromophore destruction will allow multiple treatment of a certain subset of hair follicles in order to get more complete destruction of this subset of hair follicles.

The invention may also be used for the elimination of unsightly vascular lesions such as leg veins. The strategy for eliminating leg veins using a laser or other EMR source is based on stopping the blood flow by either coagulating the blood, causing an occlusion in the vessel, or by applying external contact pressure capable of interrupting or impeding the flow of blood in the vessel. The degree to which this occlusion is necessary is determined by the irradiation parameters and the local tissue properties and depth of the treatment region. In the case of clot induction, it is preferable to substantially destroy the proximal endothelium of the blood vessel wall, which will reduce the production of the growth factors responsible for eliminating clots in the blood stream. Thus, to successfully treat the blood vessel, the inner lining of the vessel wall should be substantially destroyed;

The treatment of offending blood vessels can be augmented by mechanical or acoustical means known to those skilled in the art to achieve a reduction in blood flow or occlusion by pinching off the blood flow, or by coagulation of the blood, forming a blockage in the flow path. To ensure that enough hemoglobin and/or blood fluid chromophores remain near the treatment site, it is preferable to stop the flow of blood without ejecting the blood from the treatment region, such as might occur if a flat plate is pressed against the skin with sufficient pressure. For permanent sealing of the blood vessel, tissue welding may be induced, preferably upon heating the vessel to the degree of destroying the epithelial layer, and by applying external pressure to press the now exposed and heated collagen layers together, allowing them to form a tissue weld bond.

Using the inventive concept of heating chromophores, but not destroying the chromophores, a process can be performed whereby the hemoglobin, water in the blood and/or the blood fluid act as the chromophores, converting, the applied radiation light into heat. As long as the chromophores used in the treatments (e.g. melanin, water, hemoglobin, blood fluid) have a higher threshold for photo-thermal damage than the tissues in the surrounding treatment volume (e.g. stem cells, collagen, epithelial vessel walls) the concept presented herein can be applied to use bulk heating originating from the light-absorbing chromophores to treat the surrounding area, thus defining a prescribed treatment volume outside of which no permanent damage will occur, even if the treatment times exceed the thermal relaxation time for the tissues in question. While radiation used for blood coagulation has generally been in the 540 nm to 580 nm range in the prior art, less epidermal heating occurs at longer wavelengths, for example 810 nm available from a diode laser. By using a pulse duration which is longer that the thermal diffusion time for the vessel, a tissue volume larger than the blood vessel is heated. This bulk heating of the blood vessel and surrounding tissue results in the uniform destruction of the vessel endothelium, decreasing growth factor and perhaps ensuring the permanent blockage, and therefore elimination, of the blood vessel.

The basic idea of this application, the use of long pulse light sources having low peak power, suggests using non-laser devices to supply light energy for performing dermatological/cosmetic procedures. It appears that concern as to scarring with long pulse optical treatments, and in particular with pulses having a duration longer than the thermal relaxation time of the target component, may not be a serious restriction, since in early clinical studies, the pain threshold for the patient seems to be reached before any serious dermal damage occurs, which can lead to scarring. Therefore, so long as the treatment is terminated when the patient reports pain, the risk of scarring is very limited. This procedure can permit safe pulse durations for a given source to be empirically determined. Fig. 11 shows one set of illustrative curves. Further, low power, long pulse treatments result in the slow heating of tissue beyond the target component, which is moderated by blood flow in the region to maintain tissue temperature in such a region below its damage threshold, even for very long pulses.

Since the procedure of this invention does not destroy the chromophores, it can be expected that a weak light source, used over an extended period of time, will be effective in tissue modification/damage/destruction. This means that light sources from incandescent bulbs (home device) to halogen lamps (home device) to the sun are potential light sources for dermatologic/cosmetic procedures. Dyes which can act as surrogate chromophores in transforming light to heat energy, for example carbon or other black chromophores for a wide spectrum light source, and/or known frequency shifting or filtering techniques can be used for selected procedures, as required.

While in the discussion above, the target has generally been damaged or destroyed by being heated to a temperature well above normal body temperature, it is also possible to achieve selective target damage or other therapeutic effect by heating the target to a temperature only several degrees above body temperature for a prolonged period to achieve localized hyperthermia. Such heating may be achieved by either direct heating of a target containing a suitable chromophore or by indirect heating of the target by heat diffusion from an absorbing chromophore, as described above. Pulse durations of more than 20 seconds might be appropriate for this type of treatment, and exposure or treatment times substantially greater, perhaps even in the range of minutes to hours, may be necessary in some instances.

For example, postfebrile temporary alopecia (i.e., hair loss at elevated temperature, for example when a patient has a high fever), is a well-known phenomenon. The critical fever temperature that can cause temporary hair loss is approximately 39˚C. This indicates a susceptibility of hair follicle cells to hyperthermia. While heating the entire body nonselectively in order to achieve hair removal may not be practical and could cause unwanted side effects, a treatment procedure where temperature in a range of a few degrees above body temperature are achieved close to the hair follicles in order to cause hair loss is an option. In order to achieve hair loss with these relatively low temperature increases, exposure in the range of minutes to hours is probably necessary. This would result in the temperature distribution within the tissue being close to a steady state profile. The low peak power/long pulse duration teachings of this invention can be used to calculate appropriate power and pulse duration for achieving hyperthermia treatments. However, with prolonged exposure, the body performs compensating mechanisms, like increased blood flow and sweating, which have to be considered to calculate appropriate parameters. It therefore may be more accurate to empiracally determine appropriate parameters using for example feedback mechanisms. For example, the onset of pain or the monitoring of skin temperature either at or close to the skin surface might be utilized to control power and duration with this regimen.

A hyperthermia regimen may also be employed by introducing an artificial chromophore into the middle part of the hair shaft, a very low power EMR source being utilized in this case in combination with very long exposure times. Hyperthermia-type treatment might also be utilized for skin resurfacing with melanin in the epidermis being used to induce hyperthermia of the papillary dermis.

Long pulse heating of a hair shaft or hair follicle can also enhance adjuvant therapies, for example PDP or other photo-excited processes for hair removal. The increased temperature in the long pulse regimen can enhance susceptibility to photochemical induced damage.

It is also known that certain biological tissue responses are triggered by a prolonged exposure of tissue to elevated heat, and that some of these biological responses may enhance the susceptibility of the tissue to further tissue damage. Thus, heat exposure during an initial treatment can result in perifollicular edema which decreases perifollicular capillary blood flow. This reduces the removal of heat due to heat convection, permitting outermost structures of the hair follicle to be more effectively heated during a subsequent long pulse treatment. Other useful biological tissue responses may also occur.

Finally, while the discussion above has been primarily described with respect to various dermatology treatments, the low peak power/long pulse duration regimens of this invention are by no means limited to the field of dermatology and may be advantageously employed in other EMR therapie. For example, such a regimen might be useful for treating ophthalmic diseases such as macular degeneration where great care must be exercised to achieve therapeutic results in the desired area of the eye without causing damage to adjacent areas such as the optic nerve. Lower energy radiation delivered over a significantly longer duration might permit the desired therapeutic effects to be achieved while reducing the danger of blindness in the eye if an undesired area is accidentally momentarily irradiated.

Attached as an Exhibit to this application is an unpublished article, the contents of which are not to be printed.

While the invention has been discussed above with respect to preferred embodiments, it is apparent that these embodiments are for purposes of illustration only, and that variations thereon will be apparent to ones skilled in the art while still remaining within the spirit and scope of the invention, which is to be defined only by the appended claims.

Although the invention can be defined as stated in the attached claims, it is to be understood that the present invention can alternatively also be defined as stated in the following embodiments:

1. A method for performing a medical procedure by applying electromagnetic radiation (EMR) to a treatment area of a patient's body which may contain at least one chromophore, such radiation being of at least one wavelength absorbed by said chromophore, having a power profile with an average power ($P_a$) and peak power ($P_p$), ($P_p$) being insufficient to cause a change in the at least one chromophore which results in significant loss of absorption, and having a duration long enough for sufficient energy for the procedure to be applied at said power profile to the treatment area.

2. A method as claimed in embodiment 1, wherein said target area has a thermal relaxation time (TRT), and wherein said duration is greater than said TRT.

3. A method as claimed in embodiment 2, wherein said duration is substantially greater than the TRT.

4. A method as claimed in embodiment 1, wherein said peak power is less than 500 watts.

5. A method as claimed in embodiment 1, wherein said EMR is continuous wave, and wherein peak power and average power for said radiation over said duration are roughly the same.

6. A method as claimed in embodiment 1, where said chromophore is heated to a temperature which is no more than approximately 110˚C so as to avoid vaporization of tissue.

7. A method as claimed in embodiment 1, wherein said procedure is hair removal, wherein said chromophore is melanin of a hair shaft in a hair follicle, and wherein said duration is sufficient for sufficient heat from said hair shaft to reach an outer root sheath of said follicle to damage stem cells located thereat.

8. A method as claimed in embodiment 1, wherein said procedure is hair removal, wherein said chromophore includes a heater in at least one follicle located in said target area, and wherein said power profile and duration are sufficient to at least damage at least one critical element of the follicle.

9. A method as claimed in embodiment 1, wherein said procedure is wrinkle removal, wherein said chromophore is melanin at the dermis-epidermis (DE) junction, and wherein said power profile and duration are sufficient to heat collagen in the patient papillary dermis sufficiently to restructure such collagen.

10. A method as claimed in embodiment 1, wherein said procedure is destructions of vascular lesions, wherein said chromophore is blood in the vascular lesion, and wherein said duration is sufficiently longer, at said power profile, than the thermal diffusion time of the vessel for bulk heating of the vessel.

11. A method as claimed in embodiment 1, wherein said wavelength is absorbed by melanin in the patient's epidermis, including at the dermis-epidermis (DE) junction, and wherein said power profile and duration are such that heat generated as a result of EMR absorption in epidermal melanin can migrate to the skin surface and be removed concurrent with irradiation, thereby controlling temperature increase in the epidermis during irradiation.

12. A method as claimed in embodiment 1, including reapplying said EMR to said treatment area with a power profile and duration selected to one of retarget the same chromophore in the treatment area and target a different chromophore in the target area.

13. Apparatus for performing a medical procedure on a treatment area of a patient's body containing at least one chromophore including a source of electromagnetic radiation (EMR) of at least one wavelength absorbed by said chromophore, an applicator applying the EMR to a treatment area of a patient's body, and controls causing the EMR applied to the target area to have a power profile with an average power ($P_a$) and peak power ($P_p$), ($P_p$) being insufficient to cause a charge in the at least one chromophore which results in significant loss of absorption, and to have a duration long enough for sufficient energy for the treatment to be applied at said power profile to the treatment area.

14. Apparatus as claimed in embodiment 13, wherein said target area has a thermal relaxation time (TRT), and wherein said duration is greater than said TRT.

15. Apparatus as claimed in embodiment 14, wherein said duration is substantially greater than the TRT.

16. Apparatus as claimed in embodiment 13, wherein said peak power is less than 500 watts.

17. Apparatus as claimed in embodiment 13, wherein said EMR is continuous wave, and wherein peak power and average power for said radiation over said duration are roughly the same.

18. Apparatus as claimed in embodiment 13, wherein said chromophore is heated to temperature which is no more than approximately 110˚C so as to avoid vaporization of tissue.

19. Apparatus as claimed in embodiment 13, wherein said procedure is hair removal, wherein said chromophore is a hair shaft in a hair follicle, and wherein said duration is sufficient for sufficient heat from said hair shaft to reach an outer root sheath of said follicle to damage stem cells located thereat.

20. Apparatus as claimed in embodiment 13, wherein said procedure is hair removal, wherein said chromophore includes a heater in at least one follicle located in said target area, and wherein said power profile and duration are sufficient to at least damage at least one critical element of the follicle.

21. Apparatus as claimed in embodiment 13, wherein said procedure is wrinkle removal, wherein said chromophore is melanin at the DE junction, and wherein said power profile and duration are sufficient to heat collagen in the papillary dermis sufficiently to restructure such collagen.

22. Apparatus as claimed in embodiment 13, wherein said procedure is destructions of vascular lesions, wherein said chromophore is blood in the vascular lesion, and wherein said duration is sufficiently longer at said power profile than the thermal diffusion time of the lesion for bulk heating of the lesion.

23. Apparatus as claimed in embodiment 13, wherein said wavelength is absorbed by melanin in the patient's epidermis, including at the dermis-epidermis (DE) junction, and wherein said power profile and duration are such that heat generated as a result of EMR absorption in epidermal melanin can migrate to the skin surface and be removed concurrent with irradiation, thereby controlling temperature increase in the epidermis during irradiation.

24. Apparatus as claimed in embodiment 23 including a mechanism which actively cools at least the portion of said skin surface over the treatment area at least substantially concurrent with application of EMR to said treatment area.

25. Apparatus as claimed in embodiment 13 including a detector for at least one patient physiological condition, said controls being responsive to said detector for controlling at least one of EMR power profile and duration.

26. Apparatus as claimed in embodiment 13, wherein said radiation source is a low peak power laser source.

27. Apparatus as claimed in embodiment 13, wherein said radiation source is a low peak power non-coherent light source.

28. A method for performing a medical procedure on a target area of a patient's body, said target area having a thermal relaxation time (TRT) and including a highly absorbent heater portion, the method including applying electromagnetic radiation (EMR) to at least the heater portion of said target area of at least one wavelength highly absorbed by said heater portion, of a duration close to or greater than said thermal relaxation time, and having a power profile sufficient over said duration to accomplish said medical procedure.

29. A method as claimed in embodiment 28 wherein said duration is significantly greater than TRT.

30. A method as claimed in embodiment 28, wherein said target area has a thermal damage time (TDT) which is the time required at said power profile for said entire target area to reach a thermal destruction temperature; and wherein the duration of said radiation is substantially equal to said TDT.

31. A method as claimed in embodiment 30, wherein said duration (t) for the EMR is equal (TDT-$\delta$), where $\delta$ is roughly the propagation time for heat from the heater portion to a non-heater portion of the target furthest from the heater.

32. A method as claimed in embodiment 30, wherein TDT = TRT $\cdot$ r (x, $\Delta$), where x is a geometrical factor and $\Delta$ is a temperature factor.

33. A method as claimed in embodiment 32, wherein x = $d_2/d_1$ where $d_1$ = size of heater portion and $d_2$ = size of

total target, and wherein $\Delta = (T_2-T_0)/(T_1-T_0)$ where $T_0$ is target/heater temperature before irradiation, $T_1$ is the heater temperature and $T_2$ is the temperature at which irreversible thermal damage of the target area generally occurs.

34. A method as claimed in embodiment 30, wherein said target is a hair follicle, and said heater portion includes a pigmented hair shaft in said follicle, TDT being the time required for heat to the thermal destruction temperature to reach an outer sheath of the follicle.

35. A method as claimed in embodiment 30, wherein said target is a blood vessel, said heater portion including blood in the vessel, and TDT being the time required for heat to the thermal destruction temperature to reach through walls of the blood vessel in a target area.

36. A method as claimed in embodiment 30, wherein said target is collagen in the patient papillary dermis, said heater portion being melanin at the dermal-epidermal (DE) junction, and TDT being the time required for heat at the thermal destruction temperature to reach a desired depth in said papillary dermis.

37. A method as claimed in embodiment 30, wherein said power profile is such that at TDT, the entire target area is at a temperature of at least the thermal destruction temperature, but substantially no tissue outside the target area is at or above the thermal destruction temperature.

38. A method as claimed in embodiment 28, including cooling the patient's skin in an area over said target area to remove heat from the patient's epidermis.

39. Apparatus for performing a medical procedure on a target area of a patient's body, said target area having a thermal relaxation time (TRT) and including a highly absorbent heater portion, including : a source of electromagnetic radiation (EMR) of at least one wavelength highly absorbed by said heater portion; an applicator for applying EMR from said source to at least the heater portion of said target area; and controls for operating at least one of said source and said applicator to apply EMR to said target area for a duration significantly greater than said thermal relaxation time and with a power profile sufficient over said duration to accomplish said medical procedure.

40. Apparatus as claimed in embodiment 39, wherein said target has a thermal damage time (TDT) which is the time required at said power profile for said entire target to reach a thermal destruction temperature; and wherein the duration of said radiation applied to said target area is substantially equal to said TDT.

41. Apparatus as claimed in embodiment 40, wherein said duration (t) during which EMR is applied to the target is equal (TDT-$\delta$), where $\delta$ is roughly the propagation time for heat from the heater portion to a non-heater portion of the target furthest from the heater portion.

42. Apparatus as claimed in embodiment 40, wherein TDT = TRT • r (x, $\Delta$), where x is a geometrical factor and $\Delta$ is a temperature factor.

43. Apparatus as claimed in embodiment 42, wherein $x = d_2/d_1$ where $d_1$ = size of heater portion and $d_2$ = size of total target, and wherein $\Delta = (T_2-T_0)/(T_1-T_0)$ where $T_0$ is target/heater temperature before irradiation, $T_1$ is the heater temperature and $T_2$ is the temperature at which irreversible thermal damage of the target area generally occurs.

44. Apparatus as claimed in embodiment 39, including a mechanism operative at least during application of EMR to said target area which cools the patient's skin in an area over said target area.

45. A method for performing a medical procedure on a target area of a patient's body, said target area including a highly absorbent heater portion, the method including applying electromagnetic radiation EMR to at least the heater portion of said target area of at least one wavelength highly absorbed by said heater portion, having a power profile which heats said heater portion to a temperature T which is greater than a thermal damage temperature for at least a portion of the target area to be damaged, but less than a collapse temperature at which said heater portion undergoes a change which results in significant loss of absorption at said at least one wavelength, and having a duration sufficient with said power profile to accomplish said medical procedure.

46. A method as claimed in embodiment 45, wherein said target has a thermal damage time (TDT) which is the time required for said entire target to reach said thermal destruction temperature at said power profile, and wherein said duration is substantially equal to TDT.

47. A method as claimed in embodiment 45, wherein at said collapse temperature, said heater portion undergoes a phase transition causing at least one of bleaching, melting, boiling, bubble formation and other destructive process.

48. A method as claimed in embodiment 45, wherein said heater portion is a naturally occurring chromophore in the patient's body.

49. A method as claimed in embodiment 45, wherein said heater portion is at least in part an artificial chromophore introduced to said target area.

50. A method as claimed in embodiment 45, wherein said medical procedure is hair removal, wherein said heater portion includes a hair shaft in a hair follicle which hair shaft has a collapse temperature of up to 250°C, and wherein said duration is 0.5 ms to 20 sec.

51. A method as claimed in embodiment 45, wherein said medical procedure is removal of vascular lesions, and wherein said duration is 0.5 ms to 20 sec.

52. A method as claimed in embodiment 45, wherein said medical procedure is collagen remodeling and wherein said duration is 100 ms to 20 sec.

53. A method as claimed in embodiment 45 including repeating said applying step at least one additional time to further damage at least selected portions of said target area.

54. A method as claimed in embodiment 53 wherein heaters in a target area are not uniform and therefore have different collapse temperatures, wherein, during a selected performance of said applying step, portions of said target area heated by a heater are treated without exceeding the thermal collapse temperature of the heater, and wherein, during subsequent performance of the applying step, the thermal collapse temperature for said heaters is exceeded without exceeding the thermal collapse temperature of heaters heating portions of the target area for which treatment is not completed.

55. A method as claimed in embodiment 45 wherein said power profile is such that the temperature of said heater is substantially constant for said duration.

56. A method as claimed in embodiment 45 wherein said power profile and duration are such as to result in hyperthermia in said target area to accomplish said medical procedure.

57. Apparatus for performing a medical procedure on a target area of a patient's body, said target area including a highly absorbent heater portion, including: a source of electromagnetic radiation (EMR) of at least one wavelength highly absorbed by said heater portion, an applicator applying said radiation to at least the heater portion of said target area, and controls for at least one of said source and said applicator to cause said EMR applied to the target area to have an power profile which heats said heater to a temperature T which is greater than a thermal damage temperature for at least a portion of the target to be damaged, but less than a collapse temperature at which said heater portion undergoes a change which results in significant loss of absorption at said at least one wavelength, and of a duration sufficient with said power profile to accomplish said dermatology procedure.

58. Apparatus as claimed in embodiment 57, wherein said target has a thermal damage time (TDT) which is required for said entire target to reach said thermal destruction temperature at said power profile, and wherein said duration is substantially equal to TDT.

59. Apparatus as claimed in embodiment 57, wherein said EMR source is an optical radiation source.

60. Apparatus as claimed in embodiment 57, wherein said power profile is such the temperature of said heater is substantially constant for said duration.

61. A method for performing a medical procedure on a target area of a patient's body, said target area including at least one absorbent heater portion and at least one nonheater portion having weak absorption to no absorption, said at least one non-heater portion being spaced to varying degrees from said at least one heater portion, the method including applying electromagnetic radiation (EMR) to at least heater portions of said target area of at least one wavelength highly absorbed by said heater portions, having a power profile which heats said heater portions to a temperature T which is greater than a thermal damage temperature for the portions of the target area to be damaged, but less than the collapse temperature at which said heater portions undergo a change which results in significant loss of absorption at said at least one wavelength, and of a duration sufficient with said energy profile to permit the heating of substantially all of said target area from said heater portions to a temperature sufficient to accomplish said medical procedure.

62. A method as claimed in embodiment 61, wherein the temperature to which substantially all of said target area is heated is at least equal to a thermal destruction temperature for said target area.

63. A method as claimed in embodiment 61, wherein there are a plurality of target areas in an aperture being irradiated by said EMR, each of said areas being of a size $d_2$, and centers of said target areas being spaced by a distance $d_3$, and wherein the ratio of fluence $F_{NS}$ at which damage outside a target area occurs to fluence FS at which selective damage of a complete target area occurs is $F_{NS}/F_S = (d_3/d_2)^n$, wherein n is dependent on target shape.

64. A method for performing a medical procedure by applying electromagnetic radiation (EMR) of appropriate wavelength to a treatment area of a patient's body, such wavelength being absorbed by melanin in the patient's epidermis, including at the dermis-epidermis (DE) junction, and wherein said EMR has a power profile and duration which are sufficient to effect the medical procedure and are such that heat generated as a result of EMR absorption in epidermal melanin can migrate to the skin surface and be removed concurrent with irradiation, thereby controlling temperature increase in the epidermis during irradiation.

65. A method as claimed in embodiment 64, including actively cooling said skin surface at least during said duration.

66. Apparatus for performing a medical procedure on a patient's body including :

a source of electromagnetic radiation (EMR) of a wavelength appropriate for said procedure, said wavelength being absorbed by melanin in the patient's epidermis, including at the dermis-epidermis (DE) junction, an applicator for applying the EMR to a treatment area of the patient's body, and controls for at least one of said source and said applicator which cause said EMR to be applied with a power profile and a duration to effect said medical procedure and which are such that heat generated as a result of EMR absorption in epidermal melanin can migrate to the skin surface and be removed concurrent with irradiation, thereby controlling temperature increase in the epidermis during irradiation.

67. Apparatus as claimed in embodiment 66, wherein said source is a source of optical radiation.

68. Apparatus as claimed in embodiment 66, including a mechanism for actively cooling said skin surface at least during said EMR duration, facilitating the removal of heat therefrom.

69. A method for performing a medical procedure on a target area of a patient's body, said target area including a highly absorbent heater portion, the method including applying electromagnetic radiation (EMR) to at least the heater portion of said target area of at least one wavelength highly absorbed by said heater portion, having a power profile which heats said heat portion to a temperature T, said applying step having a duration sufficient with said power profile to accomplish said medical procedure, and said power profile resulting in the temperature T of said heater being substantially constant during said durations.

70. A method as claimed in embodiment 69, wherein said power profile results in optical power decreasing substantially exponentially during said duration.

71. Apparatus for performing a medical procedure on a target area of a patient's body, said target area including a highly absorbent heater portion, including:

a source of electromagnetic radiation (EMR) of at least one wavelength highly absorbed by said heater portion, an applicator applying said radiation to at least the heater portion of said target area, and controls for at least one of said source and said applicator to cause said EMR applied to the target area to have an power profile which heats said heater to a temperature T, said EMR having a duration sufficient with said power profile to accomplish said medical procedure, and said power profile resulting in the temperature T of said heater being substantially constant during said durations.

72. An apparatus as claimed in embodiment 71, wherein said power profile results in optical power decreasing substantially exponentially during said duration.

**Claims**

1. A method for performing a non-surgical dermatological treatment on a target area of a patient's skin, comprising:

   applying optical electromagnetic radiation (EMR) having at least one wavelength being absorbable by a chromophore in the skin, the EMR having a peak power density less than about 500 $W/cm^2$, the EMR having a pulse duration long enough for sufficient energy to be applied to the target area to effect the dermatological treatment.

2. The method of claim 1, wherein the pulse duration is greater than the thermal relaxation time (TRT) of the target area.

3. The method of claim 2, wherein said target area has a thermal damage time (TDT) which is the time required for said entire target area to reach said thermal destruction temperature, and wherein said duration is substantially equal to TDT.

4. The method of claim 1, wherein the EMR is effective to heat the target area to a temperature greater than about 70° C.

5. The method of claim 1, wherein EMR is effective to heat the chromophore to a temperature less than about 110°C.

6. The method of claim 1, wherein the chromophore is a naturally occurring chromophore in the patient's body.

7. The method of claim 1, wherein the chromophore is an artificial chromophore applied to the patient's skin.

8. The method of claim 1, wherein the peak power density is insufficient to cause a change in the at least one chromophore which results in significant loss of absorption.

9. The method of claim 1, wherein the peak power density is greater than about 100 $W/cm^2$.

10. The method of claim 1, wherein the EMR has a fluence below about 150 Joules/$cm^2$, optionally, about 50 Joules/$cm^2$.

11. The method of claim 1, wherein the EMR exhibits at least one wavelength in a range of about 0.4 microns to about 1.5 microns or in a range of about 0.95 microns to about 1.9 microns or in a range of about 2.1 microns to about 2.4 microns.

12. The method of claim 1, wherein the EMR is suitable for the treatment of sebaceous glands.

13. The method of claim 11, wherein an artificial chromophore is introduced in the sebaceous gland, and wherein the pulse duration is sufficient to heat an outer sheath of the sebaceous gland.

14. The method of claim 1, wherein the EMR is suitable for one of hair removal, wrinkle removal, or the treatment of vascular lesions.

15. The method of claim 1, further comprising actively cooling said skin surface at least during said pulse duration.

FIG. 1

FIG. 2

FIG. 3a

FIG. 3b

FIG. 3c

FIG. 3d

FIG. 4a

FIG. 4b

FIG. 4c

FIG. 5c

$T_1 = 100°C$

$T_2 = 70°C$

$T_0 = 36°C$

TRT = 27.5ms
TDT = 1.6s

Temperature, °C

r,mm

FIG. 5a

FIG. 5b

EP 2 316 372 A1

EP 2 316 372 A1

FIG. 6a

FIG. 6b

EP 2 316 372 A1

FIG. 6c

EP 2 316 372 A1

FIG. 7

TRT$_{target}$ = 27.39ms

TRT$_{heater}$ = 3.04ms

TDT = 0.357s (100°C)

TDT = 21.53ms (230°C)

$T_1$ = 230°C/100°C

$T_2$ = 70°C

$T_0$ = 36°C

Temperature, °C

r,mm

Temperature profile

FIG. 8

EP 2 316 372 A1

FIG. 9a

FIG. 9b

FIG. 9c

FIG. 9d

FIG. 10a

Hair shaft diameter, micron
Low density of hairs

FIG. 10b

Hair shaft diameter, micron
Medium density of hairs

FIG. 10c

Hair shaft diameter, micron
High density of hairs

FIG. 11

FIG. 12

$y = d_3/d_2$

FIG. 13a

FIG. 13b

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/24507 A (THERMOLASE CORP) 11 June 1998 (1998-06-11) * the whole document * * page 50, line 8 - page 56, line 29 * * page 95, line 11 - page 101, line 20 * ----- | 1-15 | INV. A61B18/20 |
| X | WO 99/29243 A (THERMOLASE CORP) 17 June 1999 (1999-06-17) * the whole document * * page 30, line 8 - line 22 * * page 36, line 5 - line 14 * * page 49, line 23 - page 51, line 30 * ----- | 1 | |
| X | WO 98/51235 A (GEN HOSPITAL CORP ;PALOMAR MEDICAL TECHNOLOGIES I (US)) 19 November 1998 (1998-11-19) * the whole document * * page 16, line 6 - line 12 * ----- | 1 | |
| A | US 5 968 034 A (FULLMER DAVID J ET AL) 19 October 1999 (1999-10-19) * the whole document * ----- | 1-15 | TECHNICAL FIELDS SEARCHED (IPC) A61B |

The present search report has been drawn up for all claims

| Place of search | Date of completion of the search | Examiner |
|---|---|---|
| Munich | 25 February 2011 | Schöffmann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 10 01 2155

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2011

| Patent document cited in search report | | Publication date | Patent family member(s) | | Publication date |
|---|---|---|---|---|---|
| WO 9824507 | A | 11-06-1998 | AU | 5375298 A | 29-06-1998 |
| | | | GB | 2335147 A | 15-09-1999 |
| | | | US | 6050990 A | 18-04-2000 |
| WO 9929243 | A | 17-06-1999 | AU | 3908099 A | 28-06-1999 |
| | | | GB | 2335603 A | 29-09-1999 |
| WO 9851235 | A | 19-11-1998 | AU | 7568698 A | 08-12-1998 |
| | | | DE | 69825447 D1 | 09-09-2004 |
| | | | DE | 69825447 T2 | 15-09-2005 |
| | | | DK | 0991372 T3 | 06-12-2004 |
| | | | EP | 1433430 A2 | 30-06-2004 |
| | | | EP | 0991372 A2 | 12-04-2000 |
| | | | ES | 2226133 T3 | 16-03-2005 |
| | | | JP | 4056091 B2 | 05-03-2008 |
| | | | JP | 2002506362 T | 26-02-2002 |
| | | | JP | 4314353 B2 | 12-08-2009 |
| | | | JP | 2003126277 A | 07-05-2003 |
| | | | JP | 2008023355 A | 07-02-2008 |
| | | | US | 6273884 B1 | 14-08-2001 |
| | | | US | 2003055414 A1 | 20-03-2003 |
| | | | US | 2006287646 A1 | 21-12-2006 |
| | | | US | 6511475 B1 | 28-01-2003 |
| | | | US | 2010228326 A1 | 09-09-2010 |
| US 5968034 | A | 19-10-1999 | AU | 742982 B2 | 17-01-2002 |
| | | | AU | 8166798 A | 04-01-1999 |
| | | | WO | 9858592 A1 | 30-12-1998 |
| | | | US | 5885274 A | 23-03-1999 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60177943 B
- US 60235814 B

- US 09078055 B